# EUROPEAN PATENT SPECIFICATION

(11) **EP 1 245 677 B1**
(45) Date of publication and mention of the grant of the patent: **27.12.2006**
(21) Application number: 01303030.9
(22) Date of filing: 30.03.2001
(51) Int. Cl.: C12N 15/31, C12N 15/62, C12N 15/70, C07K 14/30, A61K 31/711, A61K 39/02, G01N 33/535

(54) **Nucleic acids and proteins of the mycoplasma hyopneumoniae mhp3 gene and uses thereof**
Nukleinsäuren und Proteine des mph3 Gens aus Mycoplasma hypopneumoniae und deren Verwendung
Antigène MHP3 de Mycoplasma hyopneumoniae, gène le codant et leur utilisations

(43) Date of publication of application: 02.10.2002
(73) Proprietor: Pfizer Products Inc., Groton, Connecticut 06340 (US)
(72) Inventor: King, Kendall Wayne, Groton, Connecticut 06340 (US); Madura, Rebecca Anne, Groton, Connecticut 06340 (US); Rosey, Everett Lee, Groton, Connecticut 06340 (US)
(74) Representative: Hayles, James Richard

(56) References cited:
- EP-A- 1 090 995
- WO-A-96/28472
- US-A- 5 252 328

### SEQUENCE LISTING

<110> Pfizer Products Inc
<120> NUCLEIC ACIDS AND PROTEINS OF THE MYCOPLASMA PNEUMONIAE mhp3 GENE AND USES THEREOF
<130> PC10555
<140>
   <141>
   <150>
   <151>
<160> 41
<170> PatentIn Ver. 2.1
<210> 1
   <211> 1692
   <212> DNA
   <213> Mycoplasma hyopneumoniae
<400> 1
   gtttttgaat ataatagaaa atgtaaaata aaaattaatt tattaaaaaa taattgaaag 60
   tcatcgtaat taaaacaatt aattaggaga acaactatga aaaaaaagat aaaatgaaat 120
   aaatttcttg gcttaggctt agtttttccg ctttcagcaa tcgcgacaat ctctgccgga 180
   tgttgggata aagaaacaac taaagaagaa aaatcagccg ataatcaaaa taagcaaatc 240
   actgatgtct caaaaatttc aggactagtt aatgaacgaa aatccgaaat tatggccgca 300
   aaagctgatg caaacaaaca ttttgggcta aatatggcaa ttgtaaccgc tggtggaacg 360
   gtaaatgata attcatttaa ccaatcaagt tgagaggcaa ttcaacaact tggcgctctt 420
   actggaggtg agattacttc agtagatagt tcaactgctg aacttgaagg aaaatatagc 480
   tcacttgcta ataccaacaa aaatgtttga gtactttctg gttttcaaca cggtgatgcg 540
   ttcacaagat gattaaaaat ccctgaaaat aagcaattat ttactgaaaa aaatattatc 600
   atactcggaa ttgactgaac tgatactgaa aatgtaattc caacaggtcg atatattaat 660
   ttaacctata aaactgaaga agccggatga cttgcaggat atgcgaatgc ttcctttttg 720
   gcaaaaaaat tcccaagtga tccaactaaa agatcagcaa ttgttatcgg tggtgggatt 780
   tcgccagctg taactgattt tatcgctggt tatctagccg gaattaaagc ttgaaatcta 840
   aaaaattctg ataaaaaaac aaagataaca actgataaaa tcgagataaa tcttgggttt 900
   gatgttcaag atacttcaac aaaagaaaga cttgaacaaa ttgcttcaaa agataaacct 960
   tcaacactat tagctgtcgc tggaccactt actgaaattt tctcggatat aatcgcaaac 1020
   caaaatgatc gttatctcat tggtgttgac accgaccaat cacttgttta tacaaaaact 1080
   aaaaataaat ttttcacctc aattttgaaa aatttaggtt actccgtttt cagcgttctt 1140
   agtgatttat ataccaaaaa atcaaattca agaaatttag ccggctttga atttggtaaa 1200
   aaaagtgcaa ccgtttatct tggaattaaa gacaggtttg tcgatattgc tgatacttct 1260
   ttagaaggca atgataaaaa actcgcaact gaagccattt ctgaagctaa aaaagaattt 1320
   gaagaaaaaa ctaagacaat tcctgccgaa gaagttcgta aaactttaga aattccggaa 1380
   atgcctgata aacaacctga taagcaacag gaaagcttag acaaactaat taccgatatt 1440
   aataaaaatt aagtaagaaa aaataacaat tttttaacat tatatctttt tttagagatt 1500
   aattttcttc taatttagtt taatttaata taaaattata ttaaattaaa aaaataaaaa 1560
   atccggacta tttttgttcc ggatttttta tttttgtgtt actatttaat ataatgataa 1620
   atcaggatta tgcaattgaa tttattcaag tctcgaaaaa atttggcagt ttttatgcca 1680
   attacaaaat ag                                                    1692
<210> 2
   <211> 451
   <212> PRT
   <213> Mycoplasma hyopneumoniae
<400> 2
   Met Lys Lys Lys Ile Lys Trp Asn Lys Phe Leu Gly Leu Gly Leu Val
   1               5                   10                  15
   Phe Pro Leu Ser Ala Ile Ala Thr Ile Ser Ala Gly Cys Trp Asp Lys
               20                  25                  30
   Glu Thr Thr Lys Glu Glu Lys Ser Ala Asp Asn Gln Asn Lys Gln Ile
           35                  40                  45
   Thr Asp Val Ser Lys Ile Ser Gly Leu Val Asn Glu Arg Lys Ser Glu
       50                  55                  60
   Ile Met Ala Ala Lys Ala Asp Ala Asn Lys His Phe Gly Leu Asn Met
   65                  70                  75                  80
   Ala Ile Val Thr Ala Gly Gly Thr Val Asn Asp Asn Ser Phe Asn Gln
                   85                  90                  95
   Ser Ser Trp Glu Ala Ile Gln Gln Leu Gly Ala Leu Thr Gly Gly Glu
               100                 105                 110
   Ile Thr Ser Val Asp Ser Ser Thr Ala Glu Leu Glu Gly Lys Tyr Ser
           115                 120                 125
   Ser Leu Ala Asn Thr Asn Lys Asn Val Trp Val Leu Ser Gly Phe Gln
       130                 135                 140
   His Gly Asp Ala Phe Thr Arg Trp Leu Lys Ile Pro Glu Asn Lys Gln
   145                 150                 155                 160
   Leu Phe Thr Glu Lys Asn Ile Ile Ile Leu Gly Ile Asp Trp Thr Asp
                   165                 170                 175
   Thr Glu Asn Val Ile Pro Thr Gly Arg Tyr Ile Asn Leu Thr Tyr Lys
               180                 185                 190
   Thr Glu Glu Ala Gly Trp Leu Ala Gly Tyr Ala Asn Ala Ser Phe Leu
           195                 200                 205
   Ala Lys Lys Phe Pro Ser Asp Pro Thr Lys Arg Ser Ala Ile Val Ile
       210                 215                 220
   Gly Gly Gly Ile Ser Pro Ala Val Thr Asp Phe Ile Ala Gly Tyr Leu
   225                 230                 235                 240
   Ala Gly Ile Lys Ala Trp Asn Leu Lys Asn Ser Asp Lys Lys Thr Lys
                   245                 250                 255
   Ile Thr Thr Asp Lys Ile Glu Ile Asn Leu Gly Phe Asp Val Gln Asp
               260                 265                 270
   Thr Ser Thr Lys Glu Arg Leu Glu Gln Ile Ala Ser Lys Asp Lys Pro
           275                 280                 285
   Ser Thr Leu Leu Ala Val Ala Gly Pro Leu Thr Glu Ile Phe Ser Asp
       290                 295                 300
   Ile Ile Ala Asn Gln Asn Asp Arg Tyr Leu Ile Gly Val Asp Thr Asp
   305                 310                 315                 320
   Gln Ser Leu Val Tyr Thr Lys Thr Lys Asn Lys Phe Phe Thr Ser Ile
                   325                 330                 335
   Leu Lys Asn Leu Gly Tyr Ser Val Phe Ser Val Leu Ser Asp Leu Tyr
               340                 345                 350
   Thr Lys Lys Ser Asn Ser Arg Asn Leu Ala Gly Phe Glu Phe Gly Lys
           355                 360                 365
   Lys Ser Ala Thr Val Tyr Leu Gly Ile Lys Asp Arg Phe Val Asp Ile
       370                 375                 380
   Ala Asp Thr Ser Leu Glu Gly Asn Asp Lys Lys Leu Ala Thr Glu Ala
   385                 390                 395                 400
   Ile Ser Glu Ala Lys Lys Glu Phe Glu Glu Lys Thr Lys Thr Ile Pro
                   405                 410                 415
   Ala Glu Glu Val Arg Lys Thr Leu Glu Ile Pro Glu Met Pro Asp Lys
               420                 425                 430
   Gln Pro Asp Lys Gln Gln Glu Ser Leu Asp Lys Leu Ile Thr Asp Ile
           435                 440                 445
   Asn Lys Asn
       450
<210> 3
   <211> 1269
   <212> DNA
   <213> Artificial Sequence
<220>
   <223> Description of Artificial Sequence: mhp3 manipulated for in vitro expression
<400> 3
   atgtgggata aagaaacaac taaagaagaa aaatcagccg ataatcaaaa taagcaaatc 60
   actgatgtct caaaaatttc aggactagtt aatgaacgaa aatccgaaat tatggccgca 120
   aaagctgatg caaacaaaca ttttgggcta aatatggcaa ttgtaaccgc tggtggaacg 180
   gtaaatgata attcatttaa ccaatcargt tgggaggcaa ttcaacaact tggcgctctt 240
   actggaggtg agattacttc agtagatagt tcaactgctg aacttgaagg aaaatatagc 300
   tcacttgcta ataccaacaa aaatgtttgg gtactttctg gttttcaaca cggtgatgcg 360
   ttcacaagat ggttaaaaat ccctgaaaat aagcaattat ttactgaaaa aaatattatc 420
   atactcggaa ttgactggac tgatactgaa aatgtaattc caacaggtcg atatattaat 480
   ttaacctata aaactgaaga agccggatgg cttgcaggat atgcgaatgc ttcctttttg 540
   gcaaaaaaat tcccaagtga tccaactaaa agatcagcaa ttgttatcgg tggtgggatt 600
   tcgccagctg taactgattt tatcgctggt tatctagccg gaattaaagc ttggaatcta 660
   aaaaattctg ataaaaaaac aaagataaca actgataaaa tcgagataaa tcttgggttt 720
   gatgttcaag atacttcaac aaaagaaaga cttgaacaaa ttgcttcaaa agataaacct 780
   tcaacactat tagctgtcgc tggaccactt actgaaattt tctcggatat aatcgcaaac 840
   caaaatgatc gttatctcat tggtgttgac accgaccaat cacttgttta tacaaaaact 900
   aaaaataaat ttttcacctc aattttgaaa aatttaggtt actccgtttt cagcgttctt 960
   agtgatttat ataccaaaaa atcaaattca agaaatttag ccggctttga atttggtaaa 1020
   aaaagtgcaa ccgtttatct tggaattaaa gacaggtttg tcgatattgc tgatacttct 1080
   ttagaaggca atgataaaaa actcgcaact gaagccattt ctgaagctaa aaaagaattt 1140
   gaagaaaaaa ctaagacaat tcctgccgaa gaagttcgta aaactttaga aattccggaa 1200
   atgcctgata aacaacctga taagcaacag gaaagcttag acaaactaat taccgatatt 1260
   aataatcta                                                         1269
<210> 4
   <211> 423
   <212> PRT
   <213> Artificial Sequence
<220>
   <223> Description of Artificial Sequence: mhp3 manipulated for in vitro expression
<400> 4
   Met Trp Asp Lys Glu Thr Thr Lys Glu Glu Lys Ser Ala Asp Asn Gln
    1               5                  10                  15
   Asn Lys Gln Ile Thr Asp Val Ser Lys Ile Ser Gly Leu Val Asn Glu
               20                  25                  30
   Arg Lys Ser Glu Ile Met Ala Ala Lys Ala Asp Ala Asn Lys His Phe
           35                  40                  45
   Gly Leu Asn Met Ala Ile Val Thr Ala Gly Gly Thr Val Asn Asp Asn
       50                  55                  60
   Ser Phe Asn Gln Ser Gly Trp Glu Ala Ile Gln Gln Leu Gly Ala Leu
   65                  70                  75                  80
   Thr Gly Gly Glu Ile Thr Ser Val Asp Ser Ser Thr Ala Glu Leu Glu
                   85                  90                  95
   Gly Lys Tyr Ser Ser Leu Ala Asn Thr Asn Lys Asn Val Trp Val Leu
               100                 105                 110
   Ser Gly Phe Gln His Gly Asp Ala Phe Thr Arg Trp Leu Lys Ile Pro
           115                 120                 125
   Glu Asn Lys Gln Leu Phe Thr Glu Lys Asn Ile Ile Ile Leu Gly Ile
       130                 135                 140
   Asp Trp Thr Asp Thr Glu Asn Val Ile Pro Thr Gly Arg Tyr Ile Asn
   145                 150                 155                 160
   Leu Thr Tyr Lys Thr Glu Glu Ala Gly Trp Leu Ala Gly Tyr Ala Asn
                   165                 170                 175
   Ala Ser Phe Leu Ala Lys Lys Phe Pro Ser Asp Pro Thr Lys Arg Ser
               180                 185                 190
   Ala Ile Val Ile Gly Gly Gly Ile Ser Pro Ala Val Thr Asp Phe Ile
           195                 200                 205
   Ala Gly Tyr Leu Ala Gly Ile Lys Ala Trp Asn Leu Lys Asn Ser Asp
       210                 215                 220
   Lys Lys Thr Lys Ile Thr Thr Asp Lys Ile Glu Ile Asn Leu Gly Phe
   225                 230                 235                 240
   Asp Val Gln Asp Thr Ser Thr Lys Glu Arg Leu Glu Gln Ile Ala Ser
                   245                 250                 255
   Lys Asp Lys Pro Ser Thr Leu Leu Ala Val Ala Gly Pro Leu Thr Glu
               260                 265                 270
   Ile Phe Ser Asp Ile Ile Ala Asn Gln Asn Asp Arg Tyr Leu Ile Gly
           275                 280                 285
   Val Asp Thr Asp Gln Ser Leu Val Tyr Thr Lys Thr Lys Asn Lys Phe
       290                 295                 300
   Phe Thr Ser Ile Leu Lys Asn Leu Gly Tyr Ser Val Phe Ser Val Leu
   305                 310                 315                 320
   Ser Asp Leu Tyr Thr Lys Lys Ser Asn Ser Arg Asn Leu Ala Gly Phe
                   325                 330                 335
   Glu Phe Gly Lys Lys Ser Ala Thr Val Tyr Leu Gly Ile Lys Asp Arg
               340                 345                 350
   Phe Val Asp Ile Ala Asp Thr Ser Leu Glu Gly Asn Asp Lys Lys Leu
           355                 360                 365
   Ala Thr Glu Ala Ile Ser Glu Ala Lys Lys Glu Phe Glu Glu Lys Thr
       370                 375                 380
   Lys Thr Ile Pro Ala Glu Glu Val Arg Lys Thr Leu Glu Ile Pro Glu
   385                 390                 395                 400
   Met Pro Asp Lys Gln Pro Asp Lys Gln Gln Glu Ser Leu Asp Lys Leu
                   405                 410                 415
   Ile Thr Asp Ile Asn Asn Leu
               420
<210> 5
   <211> 602
   <212> DNA
   <213> Mycoplasma hyopneumoniae
<400> 5
   atgataatat ttttttcagt aaataattgc ttattttcag ggatttttaa tcatcttgtg 60
   aacgcatcac cgtgttgaaa accagaaagt actcaaacat ttttgttggt attagcaagt 120
   gagctatatt ttccttcaag ttcagcagtt gaactatcta ctgaagtaat ctcacctcca 180
   gtaagagcgc caagttgttg aattgcctct caacttgatt ggttaaatga attatcattt 240
   accgttccac cagcggttac aattgccata tttagcccaa aatgtttgtt tgcatcagct 300
   tttgcggcca taatttcgga ttttcgttca ttaactagtc ctgaaatttt tgagacatca 360
   gtgatttgct tattttgatt atcggctgat ttttcttctt tagttgtttc tttatcccaa 420
   catccggcag agattgtcgc gattgctgaa agcggaaaaa ctaagcctaa gccaagaaat 480
   ttatttcatt ttatcttttt tttcatagtt gttctcctaa ttaattgttt taattacgat 540
   gactttcaat tattttttaa taaattaatt tttattttac attttctatt atattcaaaa 600
   ac                                                                602
<210> 6
   <211> 200
   <212> PRT
   <213> Mycoplasma hyopneumoniae
<400> 6
   Met Ile Ile Phe Phe Ser Val Asn Asn Cys Leu Phe Ser Gly Ile Phe
   1               5                   10                  15
   Asn His Leu Val Asn Ala Ser Pro Cys Trp Lys Pro Glu Ser Thr Gln
               20                  25                  30
   Thr Phe Leu Leu Val Leu Ala Ser Glu Leu Tyr Phe Pro Ser Ser Ser
           35                  40                  45
   Ala Val Glu Leu Ser Thr Glu Val Ile Ser Pro Pro Val Arg Ala Pro
       50                  55                  60
   Ser Cys Trp Ile Ala Ser Gln Leu Asp Trp Leu Asn Glu Leu Ser Phe
   65                  70                  75                  80
   Thr Val Pro Pro Ala Val Thr Ile Ala Ile Phe Ser Pro Lys Cys Leu
                   85                  90                  95
   Phe Ala Ser Ala Phe Ala Ala Ile Ile Ser Asp Phe Arg Ser Leu Thr
               100                 105                 110
   Ser Pro Glu Ile Phe Glu Thr Ser Val Ile Cys Leu Phe Trp Leu Ser
           115                 120                 125
   Ala Asp Phe Ser Ser Leu Val Val Ser Leu Ser Gln His Pro Ala Glu
       130                 135                 140
   Ile Val Ala Ile Ala Glu Ser Gly Lys Thr Lys Pro Lys Pro Arg Asn
   145                 150                 155                 160
   Leu Phe His Phe Ile Phe Phe Phe Ile Val Val Leu Leu Ile Asn Cys
                   165                 170                 175
   Phe Asn Tyr Asp Asp Phe Gln Leu Phe Phe Asn Lys Leu Ile Phe Ile
               180                 185                 190
   Leu His Phe Leu Leu Tyr Ser Lys
           195                 200
<210> 7
   <211> 14
   <212> PRT
   <213> Mycoplasma hyopneumoniae
<400> 7
   Ala Gly Xaa Trp Ala Lys Glu Thr Thr Lys Glu Glu Lys Ser
   1 5 10
<210> 8
   <211> 10
   <212> PRT
   <213> Artificial Sequence
<220>
   <223> Description of Artificial Sequence: Oligonucleotide
<400> 8
   Ala Trp Val Thr Ala Asp Gly Thr Val Asn
   1 5 10
<210> 9
   <211> 21
   <212> PRT
   <213> Artificial Sequence
<220>
   <223> Description of Artificial Sequence: Oligonucleotide
<400> 9
   Ala Ile Val Thr Ala Asp Gly Thr Val Asn Asp Asn Lys Pro Asn Gln
   1 5 10 15
   Trp Val Arg Lys Tyr
   20
<210> 10
   <211> 30
   <212> DNA
   <213> Artificial Sequence
<220>
   <223> Description of Artificial Sequence: Oligonucleotide
<400> 10
   tgytgrgcna argaracnac naargargar 30
<210> 11
   <211> 30
   <212> DNA
   <213> Artificial Sequence
<220>
   <223> Description of Artificial Sequence: Oligonucleotide
<400> 11
   tgttgagcwa aagaaacwac waaagaagaa 30
<210> 12
   <211> 27
   <212> DNA
   <213> Artificial Sequence
<220>
   <223> Description of Artificial Sequence: Oligonucleotide
<400> 12
   tgrgtnacng cngayggnac ngtnaay 27
<210> 13
   <211> 27
   <212> DNA
   <213> Artificial Sequence
<220>
   <223> Description of Artificial Sequence: Oligonucleotide
<400> 13
   tgagtwacwg cwgatggwac wgtwaat 27
<210> 14
   <211> 26
   <212> DNA
   <213> Artificial Sequence
<220>
   <223> Description of Artificial Sequence: Oligonucleotide
<400> 14
   rttnacngtn ccrtcngcng tnacyc 26
<210> 15
   <211> 26
   <212> DNA
   <213> Artificial Sequence
<220>
   <223> Description of Artificial Sequence: Oligonucleotide
<400> 15
   attsacsgts ccatcsgcsg tsactc 26
<210> 16
   <211> 21
   <212> DNA
   <213> Artificial Sequence
<220>
   <223> Description of Artificial Sequence: Oligonucleotide
<400> 16
   tttgagacat cagtgatttg c 21
<210> 17
   <211> 22
   <212> DNA
   <213> Artificial Sequence
<220>
   <223> Description of Artificial Sequence: Oligonucleotide
<400> 17
   gaacgaaaat ccgaaattat gg 22
<210> 18
   <211> 22
   <212> DNA
   <213> Artificial Sequence
<220>
   <223> Description of Artificial Sequence: Oligonucleotide
<400> 18
   ctatctactg aagaatctca cc 22
<210> 19
   <211> 20
   <212> DNA
   <213> Artificial Sequence
<220>
   <223> Description of Artificial Sequence: Oligonucleotide
<400> 19
   gtgatgccgt tcacaagatg 20
<210> 20
   <211> 21
   <212> DNA
   <213> Artificial Sequence
<220> <223> Description of Artificial Sequence: Oligonucleotide
<400> 20
   cactaagaac gctgaaaacg g 21
<210> 21
   <211> 21
   <212> DNA
   <213> Artificial Sequence
<220>
   <223> Description of Artificial Sequence: Oligonucleotide
<400> 21
   gattacaact gtaaaatcga g 21
<210> 22
   <211> 20
   <212> DNA
   <213> Artificial Sequence
<220>
   <223> Description of Artificial Sequence: Oligonucleotide
<400> 22
   ggcttcttca gttttatagg 20
<210> 23
   <211> 18
   <212> DNA
   <213> Artificial Sequence
<220>
   <223> Description of Artificial Sequence: Oligonucleotide
<400> 23
   aaactcgcaa ctgaagcc 18
<210> 24
   <211> 20
   <212> DNA
   <213> Artificial Sequence
<220>
   <223> Description of Artificial Sequence: Oligonucleotide
<400> 24
   gaaatgcctg ataaacaacc 20
<210> 25
   <211> 22
   <212> DNA
   <213> Artificial Sequence
<220>
   <223> Description of Artificial Sequence: Oligonucleotide
<400> 25
   cttcagaaat ggcttcagtt gc 22
<210> 26
   <211> 25
   <212> DNA
   <213> Artificial Sequence
<220>
   <223> Description of Artificial Sequence: Oligonucleotide
<400> 26
   gctagataac cagcgataaa atcag 25
<210> 27
   <211> 19
   <212> DNA
   <213> Artificial Sequence
<220>
   <223> Description of Artificial Sequence: Oligonucleotide
<400> 27
   tgcataatcc tgatttatc 19
<210> 28
   <211> 22
   <212> DNA
   <213> Artificial Sequence
<220>
   <223> Description of Artificial Sequence: Oligonucleotide
<400> 28
   tgaaagtcat cgtaattaaa ac 22
<210> 29
   <211> 34
   <212> DNA
   <213> Artificial Sequence
<220>
   <223> Description of Artificial Sequence: Oligonucleotide
<400> 29
   aatcggcata tgtgggataa agaaacaact aaag 34
<210> 30
   <211> 34
   <212> DNA
   <213> Artificial Sequence
<220>
   <223> Description of Artificial Sequence: Oligonucleotide
<400> 30
   ggagtaatct agattattaa tatcggtaat taag 34
<210> 31
   <211> 23
   <212> DNA
   <213> Artificial Sequence
<220>
   <223> Description of Artificial Sequence: Oligonucleotide
<400> 31
   gtttttgaat ataatagaaa atg 23
<210> 32
   <211> 28
   <212> DNA
   <213> Artificial Sequence
<220>
   <223> Description of Artificial Sequence: Oligonucleotide
<400> 32
   tttattaaaa aataattgaa agtcatcg 28
<210> 33
   <211> 28
   <212> DNA
   <213> Artificial Sequence
<220>
   <223> Description of Artificial Sequence: Oligonucleotide
<400> 33
   ctattttgta attggcataa aaactgcc 28
<210> 34
   <211> 24
   <212> DNA
   <213> Artificial Sequence
<220>
   <223> Description of Artificial Sequence: Oligonucleotide
<400> 34
   gataaaatgg aataaatttc ttgg 24
<210> 35
   <211> 21
   <212> DNA
   <213> Artificial Sequence
<220>
   <223> Description of Artificial Sequence: Oligonucleotide
<400> 35
   caggttggga ggcaattcaa c 21
<210> 36
   <211> 24
   <212> DNA
   <213> Artificial Sequence
<220>
   <223> Description of Artificial Sequence: Oligonucleotide
<400> 36
   caaaaatgtt tgggtacttt ctgg 24
<210> 37
   <211> 21
   <212> DNA
   <213> Artificial Sequence
<220>
   <223> Description of Artificial Sequence: Oligonucleotide
<400> 37
   cacaagatgg ttaaaaatcc c 21
<210> 38
   <211> 22
   <212> DNA
   <213> Artificial Sequence
<220>
   <223> Description of Artificial Sequence: Oligonucleotide
<400> 38
   ggaattgact ggactgatac tg 22
<210> 39
   <211> 22
   <212> DNA
   <213> Artificial Sequence
<220>
   <223> Description of Artificial Sequence: Oligonucleotide
<400> 39
   gccggatggc ttgcaggata tg 22
<210> 40
   <211> 24
   <212> DNA
   <213> Artificial Sequence
<220>
   <223> Description of Artificial Sequence: Oligonucleotide
<400> 40
   taaagcttgg aatctaaaaa attc 24
<210> 41
   <211> 457
   <212> PRT
   <213> Mycoplasma hyorhinis
<400> 41
   Met Asn Phe Lys Lys Ser Leu Leu Phe Leu Thr Gly Thr Ile Ser Thr
     1               5                  10                  15
   Val Ala Ser Val Ala Thr Phe Val Ser Cys Gly Glu Thr Asp Lys Glu
                20                  25                  30
   Gly Lys Ile Ile Arg Ile Phe Asp Asn Ser Phe Val Lys Asp Arg Gln
           35                  40                  45
   Ala Glu Ile Glu Lys Ala Lys Asn Phe Asp Phe Asn Thr Val Leu Leu
       50                  55                  60
   Thr Ala Gly Gly Thr Val Gln Asp Lys Ser Phe Asn Gln Ser Ile Trp
   65                  70                  75                  80
   Glu Ala Val Leu Glu His Tyr Asp Gln Ile Glu Lys Thr Thr Asn Leu
                   85                  90                  95
   Asp Arg Val Ser Gln Glu Thr Asn Asn Gln Ser Glu Leu Ile Gly Lys
               100                 105                 110
   Tyr Lys Asn Phe Leu Asn Gly Asn Lys Asn Val Trp Ile Leu Thr Gly
           115                 120                 125
   Phe Gln Gln Gly Gln Glu Phe Pro Lys Phe Leu Lys Gln Thr Asp Ser
       130                 135                 140
   Asn Gly Lys Lys Tyr Ser Asp Leu Leu Ala Glu Lys Lys Val Ile Ile
   145                 150                 155                 160
   Val Ala Val Asp Trp Asp Leu Ser Lys Glu Asp Lys Asp Leu Ile Lys
                   165                 170                 175
   Ala Gly His Phe Ile Ser Leu Leu Tyr Lys Thr Glu Glu Ala Gly Phe
               180                 185                 190
   Ile Ala Gly Tyr Ala Ser Ser Lys Phe Leu Ala Tyr Lys Phe Pro Asn
           195                 200                 205
   Asp Glu Ala Lys Arg Thr Ile Ala Pro Phe Gly Gly Gly His Gly Ala
       210                 215                 220
   Gly Val Thr Asp Phe Ile Ala Gly Phe Leu Ala Gly Ile Ala Lys Tyr
   225                 230                 235                 240
   Asn Asn Asp Asn Pro Thr Ala Lys Val Thr Ile Ser Asp Asn Asn Ile
                   245                 250                 255
   Asn Ile Asp Thr Gly Phe Ile Ser Asn Asp Lys Thr Ala Thr Phe Ile
               260                 265                 270
   Asn Gly Ile Val Asn Lys Ser Ser Leu Val Leu Pro Val Ala Gly Ser
           275                 280                 285
   Leu Thr Ser Ser Val Val Asp Ala Ile Lys Lys Ser Asn Lys Asp Thr
       290                 295                 300
   Lys Tyr Leu Ile Gly Val Asp Thr Asp Gln Ser Lys Ile Phe Ser Pro
   305                 310                 315                 320
   Ala Thr Val Phe Phe Thr Ser Ile Glu Lys His Leu Gly Arg Thr Ile
                   325                 330                 335
   Tyr Gln Val Leu Thr Asp Ile Trp Leu Lys Lys Glu Asp Ser Lys Phe
               340                 345                 350
   Leu Gly Ser Phe Arg Ser Phe Lys Leu Thr Asn Pro Ala Asn Ala Thr
           355                 360                 365
   Val Tyr Lys Gly Ile Ser Asp Asp Phe Val Gly Val Ser Asn Ser Thr
       370                 375                 380
   Val Ala Asp Ala Asp Lys Val Lys Ala Gln Glu Phe Leu Asn Glu Ala
   385                 390                 395                 400
   Thr Ala Asp Phe Lys Lys Gln Ile Gln Ala Asn Pro Thr Asn Tyr Lys
                   405                 410                 415
   Ser Val Leu Gly Ile Pro Thr Met Leu Ile Asn Asp Asn Asp Ala Lys
               420                 425                 430
   Asp Asn Glu Lys Ala Ser Leu Phe His Phe Asp Asn Trp Gln Thr Tyr
           435                 440                 445
   Trp Ala Phe His Ser Arg Phe Ile Asn
       450                 455

### SEQUENCE LISTING

<110> Pfizer Products Inc
<120> NUCLEIC ACIDS AND PROTEINS OF THE MYCOPLASMA PNEUMONIAE mhp3 GENE AND USES THEREOF
<130> PC10555
<140>
   <141>
   <150>
   <151>
<160> 41
<170> PatentIn Ver. 2.1
<210> 1
   <211> 1692
   <212> DNA
   <213> Mycoplasma hyopneumoniae
<400> 1
   gtttttgaat ataatagaaa atgtaaaata aaaattaatt tattaaaaaa taattgaaag 60
   tcatcgtaat taaaacaatt aattaggaga acaactatga aaaaaaagat aaaatgaaat 120
   aaatttcttg gcttaggctt agtttttccg ctttcagcaa tcgcgacaat ctctgccgga 180
   tgttgggata aagaaacaac taaagaagaa aaatcagccg ataatcaaaa taagcaaatc 240
   actgatgtct caaaaatttc aggactagtt aatgaacgaa aatccgaaat tatggccgca 300
   aaagctgatg caaacaaaca ttttgggcta aatatggcaa ttgtaaccgc tggtggaacg 360
   gtaaatgata attcatttaa ccaatcaagt tgagaggcaa ttcaacaact tggcgctctt 420
   actggaggtg agattacttc agtagatagt tcaactgctg aacttgaagg aaaatatagc 480
   tcacttgcta ataccaacaa aaatgtttga gtactttctg gttttcaaca cggtgatgcg 540
   ttcacaagat gattaaaaat ccctgaaaat aagcaattat ttactgaaaa aaatattatc 600
   atactcggaa ttgactgaac tgatactgaa aatgtaattc caacaggtcg atatattaat 660
   ttaacctata aaactgaaga agccggatga cttgcaggat atgcgaatgc ttcctttttg 720
   gcaaaaaaat tcccaagtga tccaactaaa agatcagcaa ttgttatcgg tggtgggatt 780
   tcgccagctg taactgattt tatcgctggt tatctagccg gaattaaagc ttgaaatcta 840
   aaaaattctg ataaaaaaac aaagataaca actgataaaa tcgagataaa tcttgggttt 900
   gatgttcaag atacttcaac aaaagaaaga cttgaacaaa ttgcttcaaa agataaacct 960
   tcaacactat tagctgtcgc tggaccactt actgaaattt tctcggatat aatcgcaaac 1020
   caaaatgatc gttatctcat tggtgttgac accgaccaat cacttgttta tacaaaaact 1080
   aaaaataaat ttttcacctc aattttgaaa aatttaggtt actccgtttt cagcgttctt 1140
   agtgatttat ataccaaaaa atcaaattca agaaatttag ccggctttga atttggtaaa 1200
   aaaagtgcaa ccgtttatct tggaattaaa gacaggtttg tcgatattgc tgatacttct 1260
   ttagaaggca atgataaaaa actcgcaact gaagccattt ctgaagctaa aaaagaattt 1320
   gaagaaaaaa ctaagacaat tcctgccgaa gaagttcgta aaactttaga aattccggaa 1380
   atgcctgata aacaacctga taagcaacag gaaagcttag acaaactaat taccgatatt 1440
   aataaaaatt aagtaagaaa aaataacaat tttttaacat tatatctttt tttagagatt 1500
   aattttcttc taatttagtt taatttaata taaaattata ttaaattaaa aaaataaaaa 1560
   atccggacta tttttgttcc ggatttttta tttttgtgtt actatttaat ataatgataa 1620
   atcaggatta tgcaattgaa tttattcaag tctcgaaaaa atttggcagt ttttatgcca 1680
   attacaaaat ag                                                    1692
<210> 2
   <211> 451
   <212> PRT
   <213> Mycoplasma hyopneumoniae
<400> 2
   Met Lys Lys Lys Ile Lys Trp Asn Lys Phe Leu Gly Leu Gly Leu Val
    1               5                   10                  15
   Phe Pro Leu Ser Ala Ile Ala Thr Ile Ser Ala Gly Cys Trp Asp Lys
               20                  25                  30
   Glu Thr Thr Lys Glu Glu Lys Ser Ala Asp Asn Gln Asn Lys Gln Ile
           35                  40                  45
   Thr Asp Val Ser Lys Ile Ser Gly Leu Val Asn Glu Arg Lys Ser Glu
       50                  55                  60
   Ile Met Ala Ala Lys Ala Asp Ala Asn Lys His Phe Gly Leu Asn Met
   65                  70                  75                  80
   Ala Ile Val Thr Ala Gly Gly Thr Val Asn Asp Asn Ser Phe Asn Gln
                   85                  90                  95
   Ser Ser Trp Glu Ala Ile Gln Gln Leu Gly Ala Leu Thr Gly Gly Glu
               100                 105                 110
   Ile Thr Ser Val Asp Ser Ser Thr Ala Glu Leu Glu Gly Lys Tyr Ser
           115                 120                 125
   Ser Leu Ala Asn Thr Asn Lys Asn Val Trp Val Leu Ser Gly Phe Gln
       130                 135                 140
   His Gly Asp Ala Phe Thr Arg Trp Leu Lys Ile Pro Glu Asn Lys Gln
   145                 150                 155                 160
   Leu Phe Thr Glu Lys Asn Ile Ile Ile Leu Gly Ile Asp Trp Thr Asp
                   165                 170                 175
   Thr Glu Asn Val Ile Pro Thr Gly Arg Tyr Ile Asn Leu Thr Tyr Lys
               180                 185                 190
   Thr Glu Glu Ala Gly Trp Leu Ala Gly Tyr Ala Asn Ala Ser Phe Leu
           195                 200                 205
   Ala Lys Lys Phe Pro Ser Asp Pro Thr Lys Arg Ser Ala Ile Val Ile
       210                 215                 220
   Gly Gly Gly Ile Ser Pro Ala Val Thr Asp Phe Ile Ala Gly Tyr Leu
   225                 230                 235                 240
   Ala Gly Ile Lys Ala Trp Asn Leu Lys Asn Ser Asp Lys Lys Thr Lys
                   245                 250                 255
   Ile Thr Thr Asp Lys Ile Glu Ile Asn Leu Gly Phe Asp Val Gln Asp
               260                 265                 270
   Thr Ser Thr Lys Glu Arg Leu Glu Gln Ile Ala Ser Lys Asp Lys Pro
           275                 280                 285
   Ser Thr Leu Leu Ala Val Ala Gly Pro Leu Thr Glu Ile Phe Ser Asp
       290                 295                 300
   Ile Ile Ala Asn Gln Asn Asp Arg Tyr Leu Ile Gly Val Asp Thr Asp
   305                 310                 315                 320
   Gln Ser Leu Val Tyr Thr Lys Thr Lys Asn Lys Phe Phe Thr Ser Ile
                   325                 330                 335
   Leu Lys Asn Leu Gly Tyr Ser Val Phe Ser Val Leu Ser Asp Leu Tyr
               340                 345                 350
   Thr Lys Lys Ser Asn Ser Arg Asn Leu Ala Gly Phe Glu Phe Gly Lys
           355                 360                 365
   Lys Ser Ala Thr Val Tyr Leu Gly Ile Lys Asp Arg Phe Val Asp Ile
       370                 375                 380
   Ala Asp Thr Ser Leu Glu Gly Asn Asp Lys Lys Leu Ala Thr Glu Ala
   385                 390                 395                 400
   Ile Ser Glu Ala Lys Lys Glu Phe Glu Glu Lys Thr Lys Thr Ile Pro
                   405                 410                 415
   Ala Glu Glu Val Arg Lys Thr Leu Glu Ile Pro Glu Met Pro Asp Lys
               420                 425                 430
   Gln Pro Asp Lys Gln Gln Glu Ser Leu Asp Lys Leu Ile Thr Asp Ile
           435                 440                 445
   Asn Lys Asn
       450
<210> 3
   <211> 1269
   <212> DNA
   <213> Artificial Sequence
<220>
   <223> Description of Artificial Sequence: mhp3 manipulated for in vitro expression
<400> 3
   atgtgggata aagaaacaac taaagaagaa aaatcagccg ataatcaaaa taagcaaatc 60
   actgatgtct caaaaatttc aggactagtt aatgaacgaa aatccgaaat tatggccgca 120
   aaagctgatg caaacaaaca ttttgggcta aatatggcaa ttgtaaccgc tggtggaacg 180
   gtaaatgata attcatttaa ccaatcargt tgggaggcaa ttcaacaact tggcgctctt 240
   actggaggtg agattacttc agtagatagt tcaactgctg aacttgaagg aaaatatagc 300
   tcacttgcta ataccaacaa aaatgtttgg gtactttctg gttttcaaca cggtgatgcg 360
   ttcacaagat ggttaaaaat ccctgaaaat aagcaattat ttactgaaaa aaatattatc 420
   atactcggaa ttgactggac tgatactgaa aatgtaattc caacaggtcg atatattaat 480
   ttaacctata aaactgaaga agccggatgg cttgcaggat atgcgaatgc ttcctttttg 540
   gcaaaaaaat tcccaagtga tccaactaaa agatcagcaa ttgttatcgg tggtgggatt 600
   tcgccagctg taactgattt tatcgctggt tatctagccg gaattaaagc ttggaatcta 660
   aaaaattctg ataaaaaaac aaagataaca actgataaaa tcgagataaa tcttgggttt 720
   gatgttcaag atacttcaac aaaagaaaga cttgaacaaa ttgcttcaaa agataaacct 780
   tcaacactat tagctgtcgc tggaccactt actgaaattt tctcggatat aatcgcaaac 840
   caaaatgatc gttatctcat tggtgttgac accgaccaat cacttgttta tacaaaaact 900
   aaaaataaat ttttcacctc aattttgaaa aatttaggtt actccgtttt cagcgttctt 960
   agtgatttat ataccaaaaa atcaaattca agaaatttag ccggctttga atttggtaaa 1020
   aaaagtgcaa ccgtttatct tggaattaaa gacaggtttg tcgatattgc tgatacttct 1080
   ttagaaggca atgataaaaa actcgcaact gaagccattt ctgaagctaa aaaagaattt 1140
   gaagaaaaaa ctaagacaat tcctgccgaa gaagttcgta aaactttaga aattccggaa 1200
   atgcctgata aacaacctga taagcaacag gaaagcttag acaaactaat taccgatatt 1260
   aataatcta                                                         1269
<210> 4
   <211> 423
   <212> PRT
   <213> Artificial Sequence
<220>
   <223> Description of Artificial Sequence: mhp3 manipulated for in vitro expression
<400> 4
   Met Trp Asp Lys Glu Thr Thr Lys Glu Glu Lys Ser Ala Asp Asn Gln
    1               5                  10                  15
   Asn Lys Gln Ile Thr Asp Val Ser Lys Ile Ser Gly Leu Val Asn Glu
                20                  25                  30
   Arg Lys Ser Glu Ile Met Ala Ala Lys Ala Asp Ala Asn Lys His Phe
           35                  40                  45
   Gly Leu Asn Met Ala Ile Val Thr Ala Gly Gly Thr Val Asn Asp Asn
       50                  55                  60
   Ser Phe Asn Gln Ser Gly Trp Glu Ala Ile Gln Gln Leu Gly Ala Leu
   65                  70                  75                  80
   Thr Gly Gly Glu Ile Thr Ser Val Asp Ser Ser Thr Ala Glu Leu Glu
                   85                  90                  95
   Gly Lys Tyr Ser Ser Leu Ala Asn Thr Asn Lys Asn Val Trp Val Leu
               100                 105                 110
   Ser Gly Phe Gln His Gly Asp Ala Phe Thr Arg Trp Leu Lys Ile Pro
           115                 120                 125
   Glu Asn Lys Gln Leu Phe Thr Glu Lys Asn Ile Ile Ile Leu Gly Ile
       130                 135                 140
   Asp Trp Thr Asp Thr Glu Asn Val Ile Pro Thr Gly Arg Tyr Ile Asn
   145                 150                 155                 160
   Leu Thr Tyr Lys Thr Glu Glu Ala Gly Trp Leu Ala Gly Tyr Ala Asn
                   165                 170                 175
   Ala Ser Phe Leu Ala Lys Lys Phe Pro Ser Asp Pro Thr Lys Arg Ser
               180                 185                 190
   Ala Ile Val Ile Gly Gly Gly Ile Ser Pro Ala Val Thr Asp Phe Ile
           195                 200                 205
   Ala Gly Tyr Leu Ala Gly Ile Lys Ala Trp Asn Leu Lys Asn Ser Asp
       210                 215                 220
   Lys Lys Thr Lys Ile Thr Thr Asp Lys Ile Glu Ile Asn Leu Gly Phe
   225                 230                 235                 240
   Asp Val Gln Asp Thr Ser Thr Lys Glu Arg Leu Glu Gln Ile Ala Ser
                   245                 250                 255
   Lys Asp Lys Pro Ser Thr Leu Leu Ala Val Ala Gly Pro Leu Thr Glu
               260                 265                 270
   Ile Phe Ser Asp Ile Ile Ala Asn Gln Asn Asp Arg Tyr Leu Ile Gly
           275                 280                 285
   Val Asp Thr Asp Gln Ser Leu Val Tyr Thr Lys Thr Lys Asn Lys Phe
       290                 295                 300
   Phe Thr Ser Ile Leu Lys Asn Leu Gly Tyr Ser Val Phe Ser Val Leu
   305                 310                 315                 320
   Ser Asp Leu Tyr Thr Lys Lys Ser Asn Ser Arg Asn Leu Ala Gly Phe
                   325                 330                 335
   Glu Phe Gly Lys Lys Ser Ala Thr Val Tyr Leu Gly Ile Lys Asp Arg
               340                 345                 350
   Phe Val Asp Ile Ala Asp Thr Ser Leu Glu Gly Asn Asp Lys Lys Leu
           355                 360                 365
   Ala Thr Glu Ala Ile Ser Glu Ala Lys Lys Glu Phe Glu Glu Lys Thr
       370                 375                 380
   Lys Thr Ile Pro Ala Glu Glu Val Arg Lys Thr Leu Glu Ile Pro Glu
   385                 390                 395                 400
   Met Pro Asp Lys Gln Pro Asp Lys Gln Gln Glu Ser Leu Asp Lys Leu
                   405                 410                 415
   Ile Thr Asp Ile Asn Asn Leu
               420
<210> 5
   <211> 602
   <212> DNA
   <213> Mycoplasma hyopneumoniae
<400> 5
   atgataatat ttttttcagt aaataattgc ttattttcag ggatttttaa tcatcttgtg 60
   aacgcatcac cgtgttgaaa accagaaagt actcaaacat ttttgttggt attagcaagt 120
   gagctatatt ttccttcaag ttcagcagtt gaactatcta ctgaagtaat ctcacctcca 180
   gtaagagcgc caagttgttg aattgcctct caacttgatt ggttaaatga attatcattt 240
   accgttccac cagcggttac aattgccata tttagcccaa aatgtttgtt tgcatcagct 300
   tttgcggcca taatttcgga ttttcgttca ttaactagtc ctgaaatttt tgagacatca 360
   gtgatttgct tattttgatt atcggctgat ttttcttctt tagttgtttc tttatcccaa 420
   catccggcag agattgtcgc gattgctgaa agcggaaaaa ctaagcctaa gccaagaaat 480
   ttatttcatt ttatcttttt tttcatagtt gttctcctaa ttaattgttt taattacgat 540
   gactttcaat tattttttaa taaattaatt tttattttac attttctatt atattcaaaa 600
   ac                                                                602
<210> 6
   <211> 200
   <212> PRT
   <213> Mycoplasma hyopneumoniae
<400> 6
   Met Ile Ile Phe Phe Ser Val Asn Asn Cys Leu Phe Ser Gly Ile Phe
    1               5                  10                  15
   Asn His Leu Val Asn Ala Ser Pro Cys Trp Lys Pro Glu Ser Thr Gln
               20                  25                  30
   Thr Phe Leu Leu Val Leu Ala Ser Glu Leu Tyr Phe Pro Ser Ser Ser
           35                  40                  45
   Ala Val Glu Leu Ser Thr Glu Val Ile Ser Pro Pro Val Arg Ala Pro
       50                  55                  60
   Ser Cys Trp Ile Ala Ser Gln Leu Asp Trp Leu Asn Glu Leu Ser Phe
   65                  70                  75                      80
   Thr Val Pro Pro Ala Val Thr Ile Ala Ile Phe Ser Pro Lys Cys Leu
                   85                  90                  95
   Phe Ala Ser Ala Phe Ala Ala Ile Ile Ser Asp Phe Arg Ser Leu Thr
               100                 105                 110
   Ser Pro Glu Ile Phe Glu Thr Ser Val Ile Cys Leu Phe Trp Leu Ser
           115                 120                 125
   Ala Asp Phe Ser Ser Leu Val Val Ser Leu Ser Gln His Pro Ala Glu
       130                 135                 140
   Ile Val Ala Ile Ala Glu Ser Gly Lys Thr Lys Pro Lys Pro Arg Asn
   145                 150                 155                 160
   Leu Phe His Phe Ile Phe Phe Phe Ile Val Val Leu Leu Ile Asn Cys
                   165                 170                 175
   Phe Asn Tyr Asp Asp Phe Gln Leu Phe Phe Asn Lys Leu Ile Phe Ile
               180                 185                 190
   Leu His Phe Leu Leu Tyr Ser Lys
           195                 200
<210> 7
   <211> 14
   <212> PRT
   <213> Mycoplasma hyopneumoniae
<400> 7
   Ala Gly Xaa Trp Ala Lys Glu Thr Thr Lys Glu Glu Lys Ser
    1               5                  10
<210> 8
   <211> 10
   <212> PRT
   <213> Artificial Sequence
<220>
   <223> Description of Artificial Sequence: Oligonucleotide
<400> 8
   Ala Trp Val Thr Ala Asp Gly Thr Val Asn
   1 5 10
<210> 9
   <211> 21
   <212> PRT
   <213> Artificial Sequence
<220>
   <223> Description of Artificial Sequence: Oligonucleotide
<400> 9
   Ala Ile Val Thr Ala Asp Gly Thr Val Asn Asp Asn Lys Pro Asn Gln
   1 5 10 15
   Trp Val Arg Lys Tyr
   20
<210> 10
   <211> 30
   <212> DNA
   <213> Artificial Sequence
<220>
   <223> Description of Artificial Sequence: Oligonucleotide
<400> 10
   tgytgrgcna argaracnac naargargar 30
<210> 11
   <211> 30
   <212> DNA
   <213> Artificial Sequence
<220>
   <223> Description of Artificial Sequence: Oligonucleotide
<400> 11
   tgttgagcwa aagaaacwac waaagaagaa 30
<210> 12
   <211> 27
   <212> DNA
   <213> Artificial Sequence
<220>
   <223> Description of Artificial Sequence: Oligonucleotide
<400> 12
   tgrgtnacng cngayggnac ngtnaay 27
<210> 13
   <211> 27
   <212> DNA
   <213> Artificial Sequence
<220>
   <223> Description of Artificial Sequence: Oligonucleotide
<400> 13
   tgagtwacwg cwgatggwac wgtwaat 27
<210> 14
   <211> 26
   <212> DNA
   <213> Artificial Sequence
<220>
   <223> Description of Artificial Sequence: Oligonucleotide
<400> 14
   rttnacngtn ccrtcngcng tnacyc 26
<210> 15
   <211> 26
   <212> DNA
   <213> Artificial Sequence
<220>
   <223> Description of Artificial Sequence: Oligonucleotide
<400> 15
   attsacsgts ccatcsgcsg tsactc 26
<210> 16
   <211> 21
   <212> DNA
   <213> Artificial Sequence
<220>
   <223> Description of Artificial Sequence: Oligonucleotide
<400> 16
   tttgagacat cagtgatttg c 21
<210> 17
   <211> 22
   <212> DNA
   <213> Artificial Sequence
<220>
   <223> Description of Artificial Sequence: Oligonucleotide
<400> 17
   gaacgaaaat ccgaaattat gg 22
<210> 18
   <211> 22
   <212> DNA
   <213> Artificial Sequence
<220>
   <223> Description of Artificial Sequence: Oligonucleotide
<400> 18
   ctatctactg aagaatctca cc 22
<210> 19
   <211> 20
   <212> DNA
   <213> Artificial Sequence
<220>
   <223> Description of Artificial Sequence: Oligonucleotide
<400> 19
   gtgatgccgt tcacaagatg 20
<210> 20
   <211> 21
   <212> DNA
   <213> Artificial Sequence
<220>
   <223> Description of Artificial Sequence: Oligonucleotide
<400> 20
   cactaagaac gctgaaaacg g 21
<210> 21
   <211> 21
   <212> DNA
   <213> Artificial Sequence
<220>
   <223> Description of Artificial Sequence: Oligonucleotide
<400> 21
   gattacaact gtaaaatcga g 21
<210> 22
   <211> 20
   <212> DNA
   <213> Artificial Sequence
<220>
   <223> Description of Artificial Sequence: Oligonucleotide
<400> 22
   ggcttcttca gttttatagg 20
<210> 23
   <211> 18
   <212> DNA
   <213> Artificial Sequence
<220>
   <223> Description of Artificial Sequence: Oligonucleotide
<400> 23
   aaactcgcaa ctgaagcc 18
<210> 24
   <211> 20
   <212> DNA
   <213> Artificial Sequence
<220>
   <223> Description of Artificial Sequence: Oligonucleotide
<400> 24
   gaaatgcctg ataaacaacc 20
<210> 25
   <211> 22
   <212> DNA
   <213> Artificial Sequence
<220>
   <223> Description of Artificial Sequence: Oligonucleotide
<400> 25
   cttcagaaat ggcttcagtt gc 22
<210> 26
   <211> 25
   <212> DNA
   <213> Artificial Sequence
<220>
   <223> Description of Artificial Sequence: Oligonucleotide
<400> 26
   gctagataac cagcgataaa atcag 25
<210> 27
   <211> 19
   <212> DNA
   <213> Artificial Sequence
<220>
   <223> Description of Artificial Sequence: Oligonucleotide
<400> 27
   tgcataatcc tgatttatc 19
<210> 28
   <211> 22
   <212> DNA
   <213> Artificial Sequence
<220>
   <223> Description of Artificial Sequence: Oligonucleotide
<400> 28
   tgaaagtcat cgtaattaaa ac 22
<210> 29
   <211> 34
   <212> DNA
   <213> Artificial Sequence
<220>
   <223> Description of Artificial Sequence: Oligonucleotide
<400> 29
   aatcggcata tgtgggataa agaaacaact aaag 34
<210> 30
   <211> 34
   <212> DNA
   <213> Artificial Sequence
<220>
   <223> Description of Artificial Sequence: Oligonucleotide
<400> 30
   ggagtaatct agattattaa tatcggtaat taag 34
<210> 31
   <211> 23
   <212> DNA
   <213> Artificial Sequence
<220>
   <223> Description of Artificial Sequence: Oligonucleotide
<400> 31
   gtttttgaat ataatagaaa atg 23
<210> 32
   <211> 28
   <212> DNA
   <213> Artificial Sequence
<220>
   <223> Description of Artificial Sequence: Oligonucleotide
<400> 32
   tttattaaaa aataattgaa agtcatcg 28
<210> 33
   <211> 28
   <212> DNA
   <213> Artificial Sequence
<220>
   <223> Description of Artificial Sequence: Oligonucleotide
<400> 33
   ctattttgta attggcataa aaactgcc 28
<210> 34
   <211> 24
   <212> DNA
   <213> Artificial Sequence
<220>
   <223> Description of Artificial Sequence: Oligonucleotide
<400> 34
   gataaaatgg aataaatttc ttgg 24
<210> 35
   <211> 21
   <212> DNA
   <213> Artificial Sequence
<220>
   <223> Description of Artificial Sequence: Oligonucleotide
<400> 35
   caggttggga ggcaattcaa c 21
<210> 36
   <211> 24
   <212> DNA
   <213> Artificial Sequence
<220>
   <223> Description of Artificial Sequence: Oligonucleotide
<400> 36
   caaaaatgtt tgggtacttt ctgg 24
<210> 37
   <211> 21
   <212> DNA
   <213> Artificial Sequence
<220>
   <223> Description of Artificial Sequence: Oligonucleotide
<400> 37
   cacaagatgg ttaaaaatcc c 21
<210> 38
   <211> 22
   <212> DNA
   <213> Artificial Sequence
<220>
   <223> Description of Artificial Sequence: Oligonucleotide
<400> 38
   ggaattgact ggactgatac tg 22
<210> 39
   <211> 22
   <212> DNA
   <213> Artificial Sequence
<220>
   <223> Description of Artificial Sequence: Oligonucleotide
<400> 39
   gccggatggc ttgcaggata tg 22
<210> 40
   <211> 24
   <212> DNA
   <213> Artificial Sequence
<220>
   <223> Description of Artificial Sequence: Oligonucleotide
<400> 40
   taaagcttgg aatctaaaaa attc 24
<210> 41
   <211> 457
   <212> PRT
   <213> Mycoplasma hyorhinis
<400> 41
   Met Asn Phe Lys Lys Ser Leu Leu Phe Leu Thr Gly Thr Ile Ser Thr
     1               5                  10                  15
   Val Ala Ser Val Ala Thr Phe Val Ser Cys Gly Glu Thr Asp Lys Glu
                20                  25                  30
   Gly Lys Ile Ile Arg Ile Phe Asp Asn Ser Phe Val Lys Asp Arg Gln
             35                  40                  45
   Ala Glu Ile Glu Lys Ala Lys Asn Phe Asp Phe Asn Thr Val Leu Leu
       50                  55                  60
   Thr Ala Gly Gly Thr Val Gln Asp Lys Ser Phe Asn Gln Ser Ile Trp
    65                  70                  75                  80
   Glu Ala Val Leu Glu His Tyr Asp Gln Ile Glu Lys Thr Thr Asn Leu
                   85                  90                  95
   Asp Arg Val Ser Gln Glu Thr Asn Asn Gln Ser Glu Leu Ile Gly Lys
               100                 105                 110
   Tyr Lys Asn Phe Leu Asn Gly Asn Lys Asn Val Trp Ile Leu Thr Gly
           115                 120                 125
   Phe Gln Gln Gly Gln Glu Phe Pro Lys Phe Leu Lys Gln Thr Asp Ser
       130                 135                 140
   Asn Gly Lys Lys Tyr Ser Asp Leu Leu Ala Glu Lys Lys Val Ile Ile
   145                 150                 155                 160
   Val Ala Val Asp Trp Asp Leu Ser Lys Glu Asp Lys Asp Leu Ile Lys
                   165                 170                 175
   Ala Gly His Phe Ile Ser Leu Leu Tyr Lys Thr Glu Glu Ala Gly Phe
               180                 185                 190
   Ile Ala Gly Tyr Ala Ser Ser Lys Phe Leu Ala Tyr Lys Phe Pro Asn
           195                 200                 205
   Asp Glu Ala Lys Arg Thr Ile Ala Pro Phe Gly Gly Gly His Gly Ala
       210                 215                 220
   Gly Val Thr Asp Phe Ile Ala Gly Phe Leu Ala Gly Ile Ala Lys Tyr
   225                 230                 235                 240
   Asn Asn Asp Asn Pro Thr Ala Lys Val Thr Ile Ser Asp Asn Asn Ile
                   245                 250                 255
   Asn Ile Asp Thr Gly Phe Ile Ser Asn Asp Lys Thr Ala Thr Phe Ile
               260                 265                 270
   Asn Gly Ile Val Asn Lys Ser Ser Leu Val Leu Pro Val Ala Gly Ser
           275                 280                 285
   Leu Thr Ser Ser Val Val Asp Ala Ile Lys Lys Ser Asn Lys Asp Thr
       290                 295                 300
   Lys Tyr Leu Ile Gly Val Asp Thr Asp Gln Ser Lys Ile Phe Ser Pro
   305                 310                 315                 320
   Ala Thr Val Phe Phe Thr Ser Ile Glu Lys His Leu Gly Arg Thr Ile
                   325                 330                 335
   Tyr Gln Val Leu Thr Asp Ile Trp Leu Lys Lys Glu Asp Ser Lys Phe
               340                 345                 350
   Leu Gly Ser Phe Arg Ser Phe Lys Leu Thr Asn Pro Ala Asn Ala Thr
           355                 360                 365
   Val Tyr Lys Gly Ile Ser Asp Asp Phe Val Gly Val Ser Asn Ser Thr
       370                 375                 380
   Val Ala Asp Ala Asp Lys Val Lys Ala Gln Glu Phe Leu Asn Glu Ala
   385                 390                 395                 400
   Thr Ala Asp Phe Lys Lys Gln Ile Gln Ala Asn Pro Thr Asn Tyr Lys
                   405                 410                 415
   Ser Val Leu Gly Ile Pro Thr Met Leu Ile Asn Asp Asn Asp Ala Lys
               420                 425                 430
   Asp Asn Glu Lys Ala Ser Leu Phe His Phe Asp Asn Trp Gln Thr Tyr
           435                 440                 445
   Trp Ala Phe His Ser Arg Phe Ile Asn
       450                 455

## Description

The *mhp3* gene codes for a protein of *Mycoplasma hyopneumoniae.* The present invention relates to nucleotides and proteins of *mhp3.* The present invention further relates to novel apoprotein antigens encoded by *mhp3* for use in vaccines to prevent and treat diseases caused by infection with *Mycoplasma hyopneumoniae,* and methods for the recombinant production of such antigens.

### BACKGROUND OF THE INVENTION

*Mycoplasma hyopneumoniae (M. hyopneumoniae)* is a bacterial pathogen that causes enzootic mycoplasmal pneumonia of swine. Enzootic mycoplasmal pneumonia is a chronic disease that results in poor food conversion, stunted growth and predisposition to secondary pulmonary infections. *M. hyopneumoniae* is easily transmitted through respiratory tract secretions and by sow-to-piglet transmission, and is highly prevalent on pig farms. Approximately 99% of US swine herds are infected, costing the swine industry about $300 million annually according to a 1991 estimate.

There are no simple tests for the detection of *M. hyopneumoniae,* nor are there effective measures against infection. Testing for the detection of *M. hyopneumoniae* has been hampered by the cross reactivity of antibodies directed against *M. hyopneumoniae* to other porcine mycoplasma species. The vaccine field for the most part has been dependent on adjuvanted membrane or whole cell preparations of *M*. *hyopneumoniae* which have not elicited significant immune responses. Additionally, cloning and recombinant expression of *M. hyopneumoniae* genes has failed to produce proteins that are sufficiently protective for commercial use in vaccines.

Due to the lack of suitable vaccines, enzootic mycoplasmal pneumonia has largely been contained by early detection and isolation of infected animals. Treatment of *M. hyopneumoniae* infected animals with antibiotics has met with limited success in curtailing the course of infection.

Thus, there exists a great need to find means of preventing the spread of this disease, either through preventing infection or cure. One approach to prevention is immunization. The ability to produce large amounts of antigenic *M. hyopneumoniae* proteins or peptides *in vitro* for use in vaccine formulations would therefore greatly advance the development of preventative vaccines.

International Patent Publication WO 96/28472 identifies six protein antigen species of *M. hyopneumoniae* at molecular weights of 46-48, 52-54, 60-64, 72-75, 90-94 and 110-114 kilodaltons, and discloses partial protein sequences of the 52-54, 60-64 and 72-75 kilodalton antigens and the full length nucleotide and amino acid sequences of the 46-48 kilodalton antigen. The 46-48 kilodalton antigen, to which we will refer hereinafter as P46, corresponds to the 44 kilodalton antigen of U.S. Patent No. 5,252,328 to Faulds and a 48 kilodalton antigen described in Lee (Lee et al., 1996, J. Chromatogr. A. 737:273-279), as determined on the basis of Faulds' and Lee's disclosures of partial peptide sequences. The gene encoding P46, *i.e. p46,* was also cloned by Futo *et al.* (1995; J. Bacteriol 177:1915-1917). Later, the same group showed that the *in vitro* expressed gene product was useful in diagnosing antibody responses to *M. hyopneumoniae* infections without cross reactivity to other *Mycoplasma* species (Futo *et al.,* 1995, J. Clin. Microbiol. 33:680-683). The sequences and diagnostic uses of the *p46* gene described by Futo *et al.* are further disclosed in European Patent Publication No. 0 475 185 A1.

The partial peptide sequence of the 60-64 kilodalton antigen of WO 96/28472 has significant homology to a protein called P102 (see below). In addition, the antigen has significant homology to a 64 kilodalton antigen disclosed by Faulds (U.S. Patent No. 5,252,328).

The 72-75 kilodalton antigen of WO 96/28472 corresponds to a 65 kilodalton protein disclosed by Wise and Kim (1987, J. Bacteriol., 169:5546-5555 and U.S. Patent No. 5,788,962) and will be referred to hereinafter as P65.

The 52-54 kilodalton antigen, to which we will refer hereinafter as Mhp3, may correspond to the 50 kilodalton integral membrane protein described by Wise and Kim (1987, J. Bacteriol., 169:5546-5555) and/or the 52 kilodalton protein species disclosed in Faulds (U.S. Patent No. 5,252,328). The 52-54 kilodalton antigen is termed hereinafter MHP3. WO 96/28472 discloses the sequences of the amino terminus of the mature protein and of an internal cyanogen bromide fragment.

The 90-94 kilodalton antigen of WO 96/28472 may correspond to the adhesin p97 as disclosed by Hsu *et al.,* which is described below.

WO 96/28472 further discloses the results of vaccine trials using the 60-64 kilodalton antigen, P46 or a combination of P65 and Mhp3. The vaccinations elicited significant protection from *M. hyopneumoniae* infection.

There are multiple reports concerning outer membrane proteins of *M*. *hyopneumoniae* in the scientific and patent literature. For example, Wise and Kim (1987, J. Bacteriol., 169:5546-5555) report that there are four integral membrane protein species in *M*. *hyopneumoniae,* named p70, p65 (P65, *supra),* p50 and p44, and that the latter three are modified by covalent lipid attachments and induce a strong humoral immune response. The protective effects of the immune response were not investigated. The gene encoding the P65 protein was cloned, and its sequences and their uses, for example in vaccines and diagnostics are described in U.S. Patent No. 5,788,962.

International Patent Publication WO 91/15593 discloses five proteins of *M*. *hyopneumoniae* of apparent molecular weights of 105, 90, 85, 70 and 43 kilodaltons. A full length sequence of the gene encoding 85 kilodalton protein (protein C) was provided, as were partial nucleotide sequences encoding the other four proteins. Vaccine trials using protein C afforded the test animals 'significant' protection against *M. hyopneumoniae.*

U.S. Patent No. 5,252,328 to Faulds discloses amino terminal sequences of immunoreactive *M. hyopneumoniae* proteins, the molecular weights of which are 36, 41, 44, 48, 64, 68, 74.5, 79, 88.5, 96 and 121 kilodaltons. Other proteins identified based on the electrophoretic mobilities but for which no protein sequences were disclosed had apparent molecular weights of 22.5, 34 and 52 kilodaltons. While U.S. Patent No. 5,252,328 proposed the use of these proteins in vaccine formulations, no results of vaccine trials were reported.

International Patent Publication WO 95/09870 discloses biochemical methods for the purification of *M*. *hyopneumoniae* adhesins, the mycoplasmal integral membrane proteins responsible for adhesion to the cilia of the host's upper respiratory epithelium. WO 95/09870 also proposes assays and uses for these proteins, for example in vaccines and diagnostics. However, no cloning of adhesin genes was reported until a gene termed p97 was cloned and its product, hereinafter "P97", shown to play a role in the organism's ability to bind to ciliated cells within the respiratory tract of *M. hyopneumoniae-*infected swine (Hsu *et al.,* 1997, J. Bacteriol. 179:1317-1323). A research paper by King *et al.* (1997; Vaccine 15:25-35) disclosed Mhp1, a 124 kilodalton adhesin that is a strain variant of P97. However, attempts to vaccinate pigs against *M. hyopneumoniae* using an GST-Mhp1 fusion protein did not result in statistically significant protection against enzootic mycoplasmal pneumonia. A 94 kilodalton variant of P97 was identified by Wilton *et al.* (1998, Microbiology 144:1931-1943). Additionally, the p97 gene was shown to be part of an operon that also encodes a second protein, termed P102, of a predicted molecular weight of approximately 102 kilodaltons (Hsu *et al.,* 1998, Gene 214:13-23). Minion and Hsu suggest the use of P102 in vaccines in the international patent publication WO 99/26664 but do not report vaccine trials.

### SUMMARY OF THE INVENTION

The present invention encompasses nucleotides and proteins of the *M. hyopneumoniae mhp3* gene. The present invention also encompasses novel apoprotein antigens encoded by the *mhp3* gene for use in vaccines to prevent and treat diseases caused by infection with *M. hyopneumoniae.* Methods for the recombinant production of apo-Mhp3 are also provided.

The invention provides a vaccine formulation comprising an antigenic or immunogenic polypeptide having an amino acid sequence comprising SEQ ID NO:4, wherein said polypeptide does not have a fatty acid acylated cysteine followed by the amino acid sequence Trp Asp Lys Glu, and does not have a C-terminal homoserine lactone, and a pharmaceutically acceptable carrier. In a preferred embodiment, the antigenic or immunogenic polypeptide is expressed as a thioredoxin fusion protein, preferably by cloning into the expression Vector pBAD/Thio-TOPO, and produced by an *E. coli* BL21 strain. In a highly preferred embodiment, the vaccine further comprises at least one polypeptide selected from the group consisting of *M. hyopneumoniae* P46, P65, P97 and P102 and fragments, variants and derivatives thereof.

The invention provides a use in the manufacture of a vaccine formulation for treating or preventing a disease or disorder in an animal caused by infection with *M. hyopneumoniae* of an antigenic or immunogenic polypeptide comprising an amino acid sequence corresponding to SEQ ID NO:4. In a preferred embodiment, the animal is a pig.

The invention further provides kits for the detection of *M. hyopneumoniae.* In one embodiment, the kit provides reagents for the detection of circulating antibodies against *M. hyopneumoniae* Mhp3 protein.

This invention provides a protein having an amino acid sequence comprising SEQ ID NO:4, wherein said protein does not have a fatty acid acylated cysteine followed by the amino acid sequence Trp Asp Lys Glu, and does not have a C-terminal homoserine lactone. In another embodiment; the protein is a fusion protein. In a further embodiment, the fusion protein is a thioredoxin fusion protein.

This invention provides any of the above proteins, which is an isolated protein. This invention also provides a composition comprising any of the above proteins and a pharmaceutically acceptable carrier. In an embodiment, the composition further comprises an adjuvant. In another embodiment, the composition further comprises at least one polypeptide selected from the group consisting of *Mycoplasma hyopneumoniae* P46, P65, P97 and P102.

This invention provides an immunogenic protein having an amino acid sequence as depicted in SEQ ID NO:4, wherein the immunogenic protein does not have a fatty acid acylated cysteine followed by the amino acid sequence Trp Asp Lys Glu, and does not have a C-terminal homoserine lactone.

This invention provides a use in the manufacture of a vaccine formulation for treating or preventing a disease or disorder in an animal caused by infection with *Mycoplasma hyopneumoniae* of a protein having an amino acid sequence comprising SEQ ID NO:4, wherein said protein does not have a fatty acid acylated cysteine followed by the amino acid sequence Trp Asp Lys Glu.

In an embodiment of any of the above uses, said animal is a pig.

This invention provides a DNA encoding in the universal genetic code a protein having an amino acid sequence comprising SEQ ID NO:4, or its complement.

In other embodiments the above DNA is operably linked to a heterologous promoter. In this invention, DNA operably linked to a heterologous promoter is isolated DNA. In yet other embodiments, any one of the above DNAs further comprises an origin of replication active in a prokaryotic cell. In other embodiments, any one of the above DNAs further comprises an origin of replication active in a eukaryotic cell.

This invention provides a host cell comprising any of the above DNAs operably linked to a heterologous promoter. In an embodiment, the host cell is *E. coli* BL21 and said DNA is the expression vector pBAD/Thio-TOPO.

This invention provides a method for the production of apo-Mhp3, said method comprising (i) growing the above cells under conditions wherein apo-Mhp3 is expressed, and (ii) recovering said protein. In an embodiment, said protein is recovered in a soluble form. In another embodiment, said protein is recovered in an insoluble form.

This invention provides a use in the manufacture of a vaccine formulation for treating or preventing a disease or disorder in an animal caused by infection with *Mycoplasma hyopneumoniae* of any of the above DNAs. In an embodiment, said animal is a pig.

This invention provides a kit comprising in at least one container a protein having an amino acid sequence comprising SEQ ID NO:4 as defined above and a statement indicating that the kit is useful for diagnosis of *M. hyopneumoniae* infection. In an embodiment, the kit further comprises an anti-pig secondary antibody, and in a further embodiment the secondary antibody is conjugated to an enzyme that catalyzes a colorimetric reaction. In a still further embodiment, the enzyme is selected from the group consisting of alkaline phosphatase and horseradish peroxidase, and in another still further embodiment, the kit further comprises reagents for a colorimetric assay.

### BRIEF DESCRIPTION OF THE FIGURES

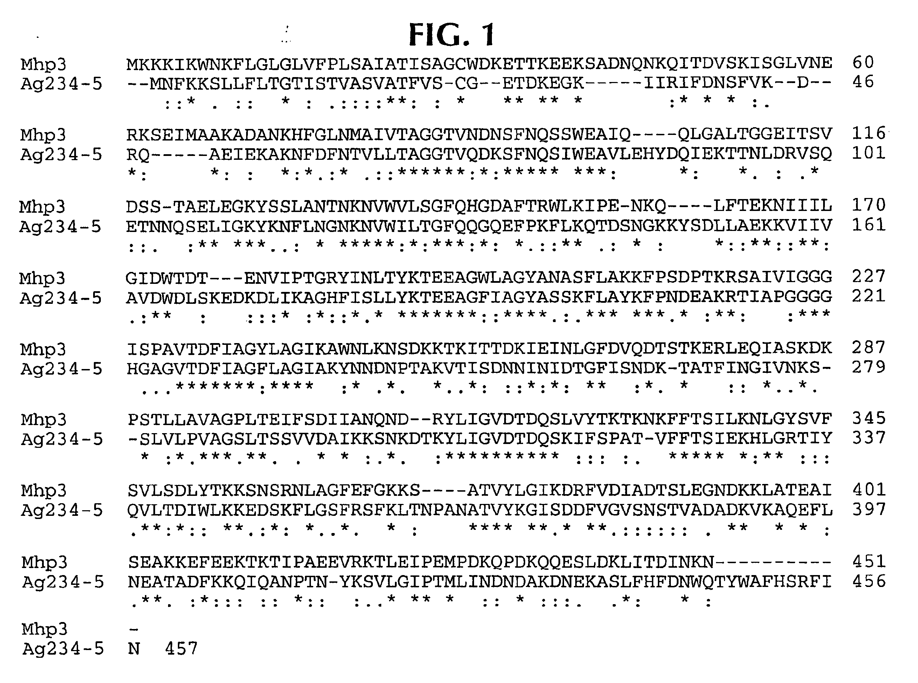

FIG. 1 is a Clustal W (1.7) sequence alignment between Mhp3 from *M. hyopneumoniae* (SEQ ID NO:2) and Ag234-5 (SEQ ID NO:41), originally thought to originate from *M. arginini* but later shown to be derived from *M. hyorhinis.* Amino acid identity is depicted by asterisks (*), highly conservative substitutions are indicated by colons (:), and conservative substitutions indicated by periods (.). Overall, the two proteins share 36.2% amino acid identity.

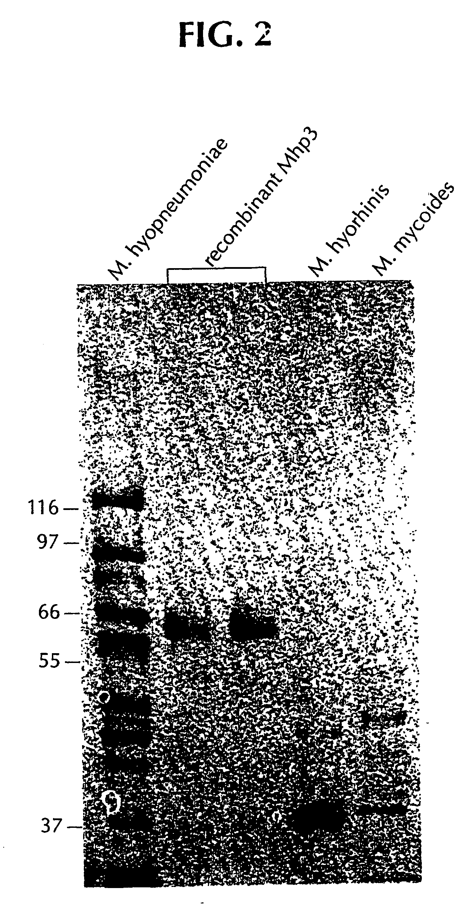

FIG. 2 is a Western blot demonstrating reactivity of antibodies from a pig experimentally challenged with *M. hyopneumoniae* against protein extracts from *M. hyopneumoniae, M. hyorhinis,* or *M. mycoides,* or against purified recombinant Mhp3.

### ABBREVIATIONS AND DEFINITIONS

The abbreviation M., where preceding the name of a species, refers to the genus *Mycoplasma.*

The term "recombinant *mhp3"* refers to a nucleic acid encoding the Mhp3 antigen in the universal genetic code. *"M. hyopneumoniae mhp3"* refers to a nucleic acid encoding the Mhp3 antigen in the *M. hyopneumoniae* genetic code. In *M. hyopneumoniae,* the codon TGA indicates a tryptophan residue rather than a translational stop codon. Thus, the recombinant *mhp3* gene differs from *M. hyopneumoniae mhp3* by having TGG codons instead of TGA codons.

The term "Mhp3" refers to a protein encoded by an *mhp3* gene.

The term "apoprotein" indicates a protein that is lacking a lipid moiety, for example by deletion or mutation of the amino acid that would function as an acceptor of said lipid moiety.

The term "ORF" indicates "open reading frame", *i.e.* the coding region of a gene.

"Percentage of sequence identity" for nucleic acids and polypeptides is determined by comparing two optimally aligned sequences over a comparison window, wherein optimal alignment provides the highest order match and may introduce additions or deletions to the test or reference sequence. The percentage identity is determined by calculating the percentage of amino acids that are identical in the test and reference sequence at a given position. Optimal sequence alignment and percentage identity may be determined manually, or more preferably by a computer algorithm including but not limited to TBLASTN, BLASTP, FASTA, TFASTA, GAP, BESTFIT, and CLUSTALW (Altschul et al., 1990, J. Mol. Biol. 215(3):403-10; Pearson and Lipman, 1988, Proc. Natl. Acad. Sci. USA 85(8):2444-8; Thompson, *et al.* 1994, Nucleic Acids Res. 22(22):4673-80; Devereux *et al.,* 1984, Nuc. Acids. Res. 12:387-395); Higgins, *et al.,* 1996, Methods Enzymol 266:383-402). Preferably, the NCBI Blast Server (http://www.ncbi.nlm.nih.gov) set at the default parameters is used to determine the percentage of sequence identity.

The term heterologous, when herein used to describe a promoter, indicates that the promoter is not native to the open reading frame whose expression it controls.

The term "isolated protein" indicates a composition of proteins in which the isolated protein comprises at least 50% by weight. More preferably, the composition comprises about 95%, and most preferably 99% by weight of the isolated protein.

The term "recovered in a soluble form" indicates that a protein or polypeptide is retrieved from the cytoplasm of the cell that expresses said protein or polypeptide.

The term "recovered in an insoluble form" indicates that a protein or polypeptide is retrieved from inclusion bodies present in the cell that expresses said protein or polypeptide.

The term "functionally equivalent", as utilized herein, refers to a protein capable of being recognized by an antibody specific to Mhp3, that is a protein capable of eliciting a substantially similar immunological response as the endogenous Mhp3 protein. Thus, an antibody raised against a functionally equivalent protein will also recognize Mhp3.

The term "immunogenicity" refers to the capability of a protein or polypeptide to elicit an immune response directed specifically against the protein or polypeptide.

The term "antigenicity" refers to the capability of a protein or polypeptide to be immunospecifically bound by an antibody to the protein or polypeptide.

The term "protection" or "protecting", as used herein with respect to a vaccine, means that the vaccine prevents or reduces the symptoms of the disease caused by the organism from which the antigen(s) used in the vaccine is derived.

The term "antibody", as used herein, refers to an immunoglobulin molecule able to bind to an antigen. Antibodies can be a polyclonal mixture or monoclonal. Antibodies can be intact immunoglobulins derived from natural sources or from recombinant sources and can be immunoreactive portions of intact immunoglobulins. Antibodies may exist in a variety of forms including, for example, Fv, Fab', F(ab')₂, as well as in single chains. Single chain antibodies, in which genes for a heavy chain and a light chain are combined into a single coding sequence, may also be used.

The term "effective amount" refers to an amount of *mhp3* nucleotide or Mhp3 polypeptide sufficient to elicit an immune response in the subject to which it is administered. The immune response may comprise, without limitation, induction of cellular and/or humoral immunity.

The term "treating or preventing *M. hyopneumoniae* infection" means to inhibit the replication of *M. hyopneumoniae* bacteria, to inhibit *M. hyopneumoniae* transmission, or to prevent *M. hyopneumoniae* from establishing itself in its host, and to alleviate the symptoms of the disease caused by *M. hyopneumoniae* infection. The treatment is considered therapeutic if there is a reduction in bacterial load, decrease in pulmonary infections and/or increase in food uptake and/or growth.

The term "pharmaceutically acceptable carrier" refers to a carrier medium that does not interfere with the effectiveness of the biological activity of the active ingredient, is chemically inert and is not toxic to the subject to whom it is administered.

The term "therapeutic agent" refers to any molecule, compound or treatment, preferably an antibacterial, that assists in the treatment of a bacterial infection or the diseases caused thereby.

### DETAILED DESCRIPTION OF THE INVENTION

As described herein, the inventor has discovered and characterized a *M*. *hyopneumoniae* gene encoding Mhp3, which is believed to be attached to the plasma membrane of *M. hyopneuomoniae* by virtue of a lipid chain covalently attached to the protein. The invention provides recombinant means of expressing Mhp3 proteins that lack a signal sequence and the necessary signal for lipid modification, thus allowing for efficient and high yield expression of Mhp3 for use in vaccines against and treatments for diseases caused by infection with *M. hyopneumoniae.*

The present invention thus encompasses proteins encoded by and nucleotide sequences of *M. hyopneumoniae mhp3.* The invention further encompasses nucleic acids encoding such proteins in the universal genetic code which are suitable for the expression of such proteins in eubacterial and eukaryotic hosts, such as *E. coli* and baculovirus, respectively.

The invention further encompasses proteins having a sequence comprising SEQ ID NO:4 and nucleic acids encoding such proteins in the universal genetic code.

The invention further encompasses uses of the proteins and/or antibodies of the invention in the manufacture of a vaccine formulation for the treatment of diseases caused by *M. hyopneumoniae.*

The invention further encompasses vaccine formulations comprising Mhp3 proteins. In certain embodiments, the vaccine formulations comprise isolated proteins selected from the group consisting of P46, P65, P97 and P102 and fragments, variants and derivatives thereof.

For clarity of disclosure, and not by way of limitation, the detailed description of the invention is divided into the following subsections which describe or illustrate certain features, embodiments or applications of the invention.

### NUCLEOTIDE SEQUENCES OF mhp3

The present invention encompasses nucleotide sequences of the *mhp3* gene and include those nucleotide sequences that encode species variants of Mhp3 as may be found in other *M. hyopneumoniae* species. A preferred embodiment of the invention encompasses the nucleotide sequences encoding an amino truncated form of the Mhp3 protein that is not modified by the covalent addition of fatty acid chains and is therefore not membrane localized. In one mode of the preferred embodiment of the invention, the 5' deletion of *mhp3* corresponding to the amino terminal of Mhp3 encompasses the nucleotide sequences encoding amino acids 2-29 of Mhp3. In other modes of the embodiment, the 5' deletion of *mhp3* corresponds to the first 30, 31-32 or 33-40 amino acids.

The invention further provides a DNA encoding in the universal genetic code a protein having an amino acid sequence comprising SEQ ID NO:4, or its complement. The invention further provides a DNA having the sequence of SEQ ID NO:3.

### mhp3-ENCODED PROTEINS AND POLYPEPTIDES

The present invention provides recombinant Mhp3 proteins. In one embodiment, the protein has an amino acid sequence comprising SEQ ID NO:4 and is not covalently linked to a fatty acid moiety. In a further embodiment, the protein is an isolated protein. The present invention also provides compositions comprising such Mhp3 proteins. In certain specific embodiments, the compositions comprise an Mhp3 protein and a pharmaceutically acceptable carrier, or an Mhp3 protein and an adjuvant. In another specific embodiment, the composition comprises at least one other protein of *M*. *hyopneumoniae* such as, but not limited to, P46, P65, P97 or P102. In other embodiments, the composition comprises an Mhp3 protein and at least one other immunogenic or antigenic polypeptide which is not a *M*. *hyopneumoniae* polypeptide and is preferably a viral, bacterial or parasitic polypeptide. Such a composition is beneficial as a combination vaccine.

Further, the Mhp3 proteins of the present invention for use in vaccine preparations are substantially pure or homogenous. Methods which are well known to those skilled in the art can be used to determine protein purity or homogeneity, such as polyacrylamide gel electrophoresis of a sample, followed by visualizing a single polypeptide band on a staining gel. Higher resolution may be determined using HPLC or other similar methods well known in the art.

The present invention encompasses polypeptides which are typically purified from host cells expressing recombinant nucleotide sequences encoding these proteins. Such protein purification can be accomplished by a variety of methods well known in the art. In one embodiment, the Mhp3 protein of the present invention is expressed as a fusion protein, for example with thioredoxin. The resulting recombinant fusion protein may be purified by affinity chromatography. In one mode of the embodiment, the Mhp3 protein is cleaved away from the heterologous moiety resulting in a substantially pure Mhp3 protein sample. Other methods may be used, see for example, the techniques described in "Methods In Enzymology", 1990, Academic Press, Inc., San Diego, "Protein Purification: Principles and practice", 1982, Springer-Verlag, New York.

### EXPRESSION SYSTEMS

The present invention encompasses expression systems, both eukaryotic and prokaryotic expression vectors, which may be used to express both truncated and full-length forms of the *mhp3* protein.

In a preferred embodiment of the invention, the nucleic acid has the sequence of SEQ ID NO:3 and encodes a protein of SEQ ID NO:4, which includes residues 30-444 of SEQ ID NO:2. The TGA codons of *M*. *hyopeumoniae* have been changed to TGG codons.

A variety of host-expression vector systems may be utilized to express the antigenic protein sequences of the invention. Such host-expression systems represent vehicles by which the coding sequences of interest may be produced and subsequently purified, but also represent cells which may, when transformed or transfected with the appropriate nucleotide coding sequences, exhibit the *mhp3* gene products of the invention *in situ.* These include but are not limited to microorganisms such as bacteria (*e.g., E. coli, B. subtilis)* transformed with recombinant bacteriophage DNA, plasmid DNA or cosmid DNA expression vectors containing *mhp3* coding sequences; yeast (*e.g*., Saccharomyces, Pichia) transformed with recombinant yeast expression vectors containing the *mhp3* gene product coding sequences; insect cell systems infected with recombinant virus expression vectors (*e.g*., baculovirus) containing the *mhp3* coding sequences; plant cell systems infected with recombinant virus expression vectors *(e.g.,* cauliflower mosaic virus, CaMV; tobacco mosaic virus, TMV) or transformed with recombinant plasmid expression vectors *(e.g.,* Ti plasmid) containing *mhp3* coding sequences; or mammalian cell systems (*e.g*., COS, CHO, BHK, 293, 3T3) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter).

In a preferred embodiment, the expression system is a bacterial system. A number of expression vectors may be advantageously selected depending upon the use intended for the *mhp3* product being expressed. For example, when a large quantity of such a protein is to be produced, for the generation of pharmaceutical compositions of Mhp3 or for raising antibodies to Mhp3, for example, vectors which direct the expression of high levels of fusion protein products that are readily purified may be desirable. Preferably, the vectors contain promoters that direct inducible gene expression. Suitable vectors include, but are not limited, to the *E. coli* expression vector pUR278 (Ruther et al., 1983, EMBO J. 2:1791), in which the *mhp3* coding sequences may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 264:5503-5509); pET vectors (Studier and Moffatt, 1986, J. Mol. Biol. 189:113; Rosenberg et al., 1987, Gene 56:125-135; the vectors are available from Novagen, Madison, Wisconsin), in which the *mhp3* coding sequence can be fused in frame to a sequence encoding multiple (*e.g*. six) histidine residues; pBAD vectors (Guzman et al., 1995, J. Bact. 177:4121-4130), using which Mhp3 can be expressed under the control of an arabinose inducible protein. pGEX vectors (Promega Corporation) may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The pGEX vectors are designed to include thrombin or factor Xa protease cleavage sites so that the cloned target gene product can be released from the GST moiety. The *mhp3* sequences can be cloned into a λ expression vector and expressed in λ⁻ bacterial strains. In a preferred mode of the embodiment, the bacterial strain is LW14, which contains a temperature sensitive λ repressor, such that protein expression from a A vector is repressed at 30°C but active at 42°C. In a highly preferred embodiment, the vector Mhp3 is expressed as a thioredoxin fusion protein, which promotes the solubility of proteins that are normally insoluble. A thioredoxin-Mhp3 fusion protein is preferably expressed by ligating an Mhp3 coding sequence into the pBAD vector pBAD/Thio-TOPO (Invitrogen Corporation, Carlsbad, California). In a highly preferred mode of the embodiment, the thioredoxin-Mhp3 fusion protein is expressed in the *E. coli* strain BL21. Other vectors can be used and are known to those skilled in the art.

### Mhp3 ANTIBODIES

According to the invention *mhp3*-encoded proteins and their derivatives and analogs may be used as an immunogens to generate antibodies which immunospecifically bind such an immunogen.

Various procedures known in the art may be used for the production of polyclonal antibodies to Mhp3. For the production of antibody, various host animals can be immunized by injection with the native Mhp3 protein, or a synthetic version, or derivative thereof, including but not limited to rabbits, mice, rats, *etc.* Various adjuvants may be used to increase the immunological response, depending on the host species (see, e.g. those used for the preparation of vaccines).

For preparation of monoclonal antibodies directed to a *mhp3*-encoded protein sequence or analog thereof, any technique which provides for the production of antibody molecules by continuous cell lines in culture may be used. For example, the hybridoma technique originally developed by Kohler and Milstein, (Kohler and Milstein 1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in *Monoclonal Antibodies and Cancer Therapy,* Alan R. Liss, Inc., pp. 77-96). In an additional embodiment of the invention, monoclonal antibodies can be produced in germ-free animals utilizing recent technology (see *e.g*., PCT/US90/02545). According to the invention, human antibodies may be used and can be obtained by using human hybridomas (Cole et al., 1983, Proc. Natl. Acad. Sci. U.S.A. 80:2026-2030) or by transforming human B cells with EBV virus *in vitro* (Cole et al., 1985, in *Monoclonal Antibodies and Cancer Therapy,* Alan R. Liss, pp. 77-96). In fact, according to the invention, techniques developed for the production of "chimeric antibodies" (Morrison et al., 1984, Proc. Natl. Acad. Sci. U.S.A. 81:6851-6855; Neuberger et al., 1984, Nature 312:604-608; Takeda et al., 1985, Nature 314:452-454) by splicing the genes from a mouse antibody molecule specific for a *mhp3-*encoded protein together with genes from a human antibody molecule of appropriate biological activity can be used; such antibodies are within the scope of this invention.

According to the invention, techniques described for the production of single chain antibodies (U.S. Patent No. 4,946,778) can be adapted to produce *mhp3* gene product-specific single chain antibodies. An additional embodiment of the invention utilizes the techniques described for the construction of Fab' expression libraries (Huse et al., 1989, Science 246:1275-1281) to allow rapid and easy identification of monoclonal Fab fragments with the desired specificity for *mhp3-*encoded proteins, derivatives, or analogs.

Antibody fragments which contain the idiotype of the molecule can be generated by known techniques. For example, such fragments include but are not limited to, the F(ab')₂ fragment which can be produced by pepsin digestion of the antibody molecule, the Fab' fragments which can be generated by reducing the disulfide bridges of the F(ab')₂ fragment, the Fab fragments which can be generated by treating the antibody molecule with papain and a reducing agent, and Fv fragments.

In the production of antibodies, screening for the desired antibody can be accomplished by techniques known in the art (*e.g*., enzyme-linked immunosorbent assay or ELISA). For example, to select antibodies which recognize a specific domain of a *mhp3-*encoded protein, one may assay generated hybridomas for a product which binds to a Mhp3 fragment containing such domain. For selection of an antibody that specifically binds a first Mhp3 homolog but which does not specifically bind a different Mhp3 homolog, one can select on the basis of positive binding to the first Mhp3 homolog and a lack of binding to the second Mhp3 homolog.

Antibodies specific to a domain of a *mhp3-*encoded protein are also provided. Antibodies specific to an epitope of a *mhp3*-encoded protein are also provided.

The foregoing antibodies can be used in methods known in the art relating to the localization and activity of the *mhp3*-encoded protein sequences of the invention, *e.g:,* measuring levels thereof in appropriate physiological samples, in diagnostic' methods, immunotherapy, *etc.*

### mhp3 KITS

The invention further provides kits for the detection of *M. hyopneumoniae.* In one embodiment, the kit provides reagents for the detection of circulating antibodies against *M. hyopneumoniae* Mhp3 protein. In certain embodiments, the kits comprise a statement indicating that the kit is useful for diagnosis of *M. hyopneumoniae* infection. Minimally, the kit comprises in at least one container a protein having an amino acid sequence comprising SEQ ID NO:4. In one mode of the embodiment, the kit further comprises an anti-pig secondary antibody. In a preferred mode of the embodiment; the secondary antibody is conjugated to an enzyme that catalyzes a colorimetric reaction, such as alkaline phosphatase or horseradish peroxidase. In a further mode of the embodiment, the kit further comprises reagents for a colorimetric assay.

### VACCINE FORMULATIONS AND METHODS OF ADMINISTRATION

Since the Mhp3 protein antigen of the present invention can be produced in large amounts, the antigen thus produced and purified has use in vaccine preparations. The Mhp3 protein also has utility in immunoassays, *e.g*., to detect or measure in a sample of body fluid from a vaccinated or potentially infected test animal the presence of antibodies to the antigen, and thus to monitor the immune response and/or to diagnose infection of the animal.

The preparation of vaccines containing an immunogenic polypeptide as the active ingredient is known to one skilled in the art.

### DETERMINATION OF VACCINE EFFICACY

The immunopotency of the Mhp3 antigen can be determined by monitoring the immune response in test animals following immunization with the Mhp3 antigen, alone or in combination with at least one other polypeptide, or by use of any immunoassay known in the art. In a preferred embodiment, said other polypeptide is selected from the group consisting of *M. hyopneumoniae* P46, P65, P97 and P102 proteins. Generation of a humoral (antibody) response and/or cell-mediated immunity may be taken as an indication of an immune response.

Methods of introducing the vaccine may include oral, intradermal, intramuscular, intraperitoneal, intravenous, subcutaneous, intranasal or any other standard routes of immunization. The immune response of the test subjects can be analyzed by various approaches such as: the reactivity of the resultant immune serum to the Mhp3 antigen, as assayed by known techniques, *e.g*., immunosorbant assay (ELISA), immunoblots, radioimmunoprecipitations, etc., or in the case where the Mhp3 antigen displays antigenicity or immunogenicity, by protection of the immunized host from infection by *M*. *hyopneumoniae* and/or attenuation of symptoms due to infection by *M. hyopneumoniae* in the immunized host.

### VACCINE FORMULATIONS

The vaccines of the invention comprise recombinant Mhp3 and/or fragments, variants and derivatives thereof. In certain embodiments, the vaccines of the inventions comprise Mhp3 and at least one other antigenic *M. hyopneumoniae* polypeptide. Suitable antigenic polypeptides for use in combination with Mhp3 include but are not limited to the P46, P65, P97 and P102 proteins and/or fragments, variants and derivatives of said polypeptides. The non-Mhp3 polypeptides of the vaccines, such as the P46, P65, P97 and P102 proteins and their fragments, variants and derivatives, may be purified from *M. hyopneumoniae* cultures or recombinantly expressed.

Suitable preparations of such vaccines further include injectables, either as liquid solutions or suspensions; solid forms suitable for solution in, or suspension in, liquid prior to injection, may also be prepared. The preparation may also be emulsified. The active immunogenic ingredients are often mixed with adjuvants which are pharmaceutically acceptable and compatible with the active ingredient.

Examples of effective adjuvants include, but are not limited to: aluminum hydroxide, N-acetyl-muramyl-L-threonyl-D-isoglutamine (thr-MDP), N-acetyl-nor-muramyl-L-alanyl-D-isoglutamine, N-acetylmuramyl-L-alanyl-D-isoglutaminyl-L-alanine-2-(1'-2'-dipalmitoyl-sn-glycero-3-hydroxyphosphoryloxy)-ethylamine.

The effectiveness of an adjuvant may be determined by measuring the induction of antibodies directed against an immunogenic polypeptide containing a Mhp3 polypeptide epitope, the antibodies resulting from administration of this polypeptide in vaccines which are also comprised of the various adjuvants.

The polypeptides may be formulated into the vaccine as neutral or salt forms. Pharmaceutically acceptable salts include the acid addition salts (formed with free amino groups of the peptide) and which are formed with inorganic acids, such as, for example, hydrochloric or phosphoric acids, or organic acids such as acetic, oxalic, tartaric, maleic, and the like. Salts formed with free carboxyl groups may also be derived from inorganic bases, such as, for example, sodium potassium, ammonium, calcium, or ferric hydroxides, and such organic bases as isopropylamine, trimethylamine, 2-ethylamino ethanol, histidine, procaine and the like.

Many methods may be used to introduce the vaccine formulations of the invention; these include but are not limited to oral, intradermal, intramuscular, intraperitoneal, subcutaneous, intranasal routes, and via scarification (scratching through the top layers of skin, e.g., using a bifurcated needle).

The subject to which the vaccine is administered is preferably an animal, most preferably a pig.

The vaccine formulations of the invention comprise an effective immunizing amount of the Mhp3 protein and a pharmaceutically acceptable carrier. Vaccine preparations comprise an effective immunizing amount of one or more antigens and a pharmaceutically acceptable carrier. Pharmaceutically acceptable carriers are well known in the art and include but are not limited to saline, buffered saline, dextrose, water, glycerol, sterile isotonic aqueous buffer, and combinations thereof. One example of such an acceptable carrier is a physiologically balanced culture medium containing one or more stabilizing agents such as stabilized, hydrolyzed proteins, lactose, etc. The carrier is preferably sterile. The formulation should suit the mode of administration.

In a specific embodiment, a lyophilized Mhp3 polypeptide of the invention is provided in a first container; a second container comprises diluent consisting of an aqueous solution of 50% glycerin, 0.25% phenol, and an antiseptic (*e.g*., 0.005% brilliant green).

Use of purified antigens as vaccine preparations can be carried out by standard methods. For example, the purified protein(s) should be adjusted to an appropriate concentration, formulated with any suitable vaccine adjuvant and packaged for use. Suitable adjuvants may include, but are not limited to: mineral gels, *e.g*., aluminum hydroxide; surface active substances such as lysolecithin; glycosides, *e.g.,* saponin derivatives such as Quil A; pluronic polyols; polyanions; non-ionic block polymers, e.g., Pluronic F-127 (B.A.S.F., USA); peptides; mineral oils, *e.g*. Montanide ISA-50 (Seppic, Paris, France), oil emulsions, *e.g*. an emulsion of mineral oil such as BayoIF/Arlacel A and water, or an emulsion of vegetable oil, water and an emulsifier such as lecithin; alum, and MDP. The immunogen may also be incorporated into liposomes, or conjugated to polysaccharides and/or other polymers for use in a vaccine formulation. In instances where the recombinant antigen is a hapten, *i.e.,* a molecule that is antigenic in that it can react selectively with cognate antibodies, but not immunogenic in that it cannot elicit an immune response, the hapten may be covalently bound to a carrier or immunogenic molecule; for instance, a large protein such as serum albumin will confer immunogenicity to the hapten coupled to it. The hapten-carrier may be formulated for use as a vaccine.

Effective doses (immunizing amounts) of the vaccines of the invention may also be extrapolated from dose-response curves derived from model test systems.

The invention also provides a pharmaceutical pack or kit comprising one or more containers comprising one or more of the ingredients of the vaccine formulations of the invention.

The present invention thus provides a method of immunizing an animal, or treating or preventing various diseases or disorders in an animal, comprising administering to the animal an effective immunizing dose of a vaccine of the present invention.

### USE OF ANTIBODIES GENERATED BY THE VACCINES OF THE INVENTION

The antibodies generated against the antigen by immunization with the Mhp3 proteins of the present invention also have potential uses in diagnostic immunoassays, passive immunotherapy, and generation of antiidiotypic antibodies.

The generated antibodies may be isolated by standard techniques known in the art (*e.g*., immunoaffinity chromatography, centrifugation, precipitation, etc.) and used in diagnostic immunoassays. The antibodies may also be used to monitor treatment and/or disease progression. Any immunoassay system known in the art, such as those listed *supra,* may be used for this purpose including but not limited to competitive and noncompetitive assay systems using techniques such as radioimmunoassays, ELISA (enzyme-linked immunosorbent assays), "sandwich" immunoassays, precipitin reactions, gel diffusion precipitin reactions, immunodiffusion assays, agglutination assays, complement-fixation assays, immunoradiometric assays, fluorescent immunoassays, protein A immunoassays and immunoelectrophoresis assays, to name but a few.

The vaccine formulations of the present invention can also be used to produce antibodies for use in passive immunotherapy, in which short-term protection of a host is achieved by the administration of pre-formed antibody directed against a heterologous organism.

In immunization procedures, the amount of immunogen to be used and the immunization schedule will be determined by a physician skilled in the art and will be administered by reference to the immune response and antibody titers of the subject.

### PACKAGING

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. Compositions comprising a compound of the invention formulated in a compatible pharmaceutical carrier may also be prepared, placed in an appropriate container, and labeled for treatment of an indicated condition.

### EXAMPLES

### Isolation of M. hyopneumoniae Chromosomal DNA

Genomic DNA from *M. hyopneumoniae* was isolated by the method of Dybvig and Alderete (Plasmid, 20:33-41; 1988), in which cells were harvested by centrifugation (10 min. at 6000g at 4°C), suspended in phosphate-buffered saline, and lysed by addition of 0.1 volume 10% sodium dodecyl sulfate. The lysate was extracted with a mixture of phenol, chloroform, and isoamyl alcohol (25:24:1) saturated with Tris-HCl. The aqueous phase was extracted with chloroform, and nucleic acids precipitated by the addition of 0.1 volume of 3M sodium acetate and 2 volumes of ice-cold ethanol. After incubation at -20°C for 1 hr, nucleic acids were recovered by centrifugation for 10 min in a microcentrifuge. Nucleic acids were resuspended in water containing 20 µg of RNase A/mL, and samples were stored at -20°C.

### Molecular Cloning of M. hyopneumoniae mhp3

Degenerate oligonucleotide primers KWK40, KWK41, KWK42, KWK43, KWK42RC, and KWK43RC (SEQ ID NOs:10-15), were designed and synthesized (Genosys Biotechnologies, Inc.; The Woodlands, TX) based upon previously identified partial amino acid sequences (SEQ ID NOs:7-9) of Mhp3 (International patent publication WO 96/28472). The polymerase chain reaction (PCR) was carried out with various combinations of these primers in a 50µL reaction volume containing 1x PCR buffer (Perkin Elmer; Foster City, CA), 2.0 mM MgCl₂, 1 µM each primer, 400 µM each deoxy-NTP, and 2.5 U AmpliTaq Gold (Perkin Elmer). Conditions for amplification consisted of denaturation at 94°C for 2 min, followed by 25 cycles of denaturation (94°C, 1 min), annealing (56°C, 1 min), and polymerization (72°C, 1 min). Using primers KWK41 (SEQ ID NO:11) and KWK42RC (SEQ ID NO:14), a fragment approximately 250 bp in length was amplified using 410 ng of *M. hyopneumoniae* strain 232 chromosomal DNA (passage n=5). The fragment was subcloned into the TA cloning site of pCR2.1-TOPO (Invitrogen; Carlsbad, CA); the ligated product was transformed into *E. coli* TOP10 cells (Invitrogen). Cloning was confirmed by dideoxynucleotide chain termination sequencing (Advanced Genetic Analysis Center; St. Paul, MN) and restriction endonuclease digestion followed by agarose gel electrophoresis.

Inverse PCR, a method used to amplify sequences flanking a core region of DNA (Ochman, H., Medhora, M.M., Garza, D., and Hartl, D.L. 1990. In "PCR Protocols: A Guide to Methods and Applications." Academic Press, San Diego, CA) was used to clone the remaining 5' and 3' ends of the gene encoding Mhp3. Pools of *M. hyopneumoniae* strain 232 (passage n=5) chromosomal DNA fragments were generated by singly digesting uncut DNA with the following restriction endonucleases: *Alul, Bam*Hl*, Eco*RV, *Eco*Rl, *Hae*lll, *Hinc*ll*, Hind*lll*, Nde*l*, Pvu*ll*, Sau*3Al, *Spe*l*, Ssp*l, *Xba*l. Following digestion, the fragments were circularized by ligation and subjected to PCR using oligonucleotide primers RAC3 (SEQ ID NO:16) and RAC4 (SEQ ID NO:17), which were designed based upon sequence from the 250 bp fragment described above. Amplification with these primers was carried out as follows: denaturation at 94°C for 2 min, followed by 40 cycles of denaturation (94°C, 1 min), annealing (55°C, 1 min), and polymerization (72°C, 2 min). The *Ssp*I-digested chromosomal DNA pool yielded an approximate 600 bp fragment which was subcloned into pCR2.1-TOPO. Sequence analysis of the cloned fragment identified the 5' end of the gene. Oligonucleotides RAC7 (SEQ ID NO:18) and RAC8 (SEQ ID NO:19) were designed based upon this sequence in order to obtain further sequence corresponding to the 3' end of *mhp3.* Conditions for amplification with these primers consisted of denaturation at 94°C for 9 min, followed by 40 cycles of denaturation (94°C, 30 sec), annealing (50°C, 30 sec), and polymerization (72°C, 1 min). When the Spel-digested DNA pool was used as template, multiple products (250 bp to >1 kb) were amplified; the mixture of fragments was cloned into pCR2.1-TOPO. Sequence analysis of the largest fragment cloned (-1.2kb) provided additional 3' sequence data. From this data, oligonucleotides RAC12 (SEQ ID NO:20) and RAC15 (SEQ ID NO:23) were designed and synthesized to be used for PCR amplification using the *Hind*III-digested DNA pool as template. Parameters consisted of denaturation at 94°C for 9 min, followed by 40 cycles of denaturation (94°C, 30 sec), annealing (55°C, 30 sec), and polymerization (72°C, 2 min), plus a final polymerization step at 72°C for 7 min. A fragment approximately 600 bp in length was amplified, cloned into pCR2.1-TOPO, and sequenced. Data obtained provided additional 3' sequence within the gene encoding Mhp3. Further PCR amplification with RAC12 (SEQ ID NO:20) and RAC15 (SEQ ID NO:23) was carried out using the *Hinc*ll-digested DNA pool as template. Conditions for amplification were denaturation at 94°C for 9 min, followed by 35 cycles of denaturation (94°C, 1 min), annealing (55°C, 1 min), and polymerization (72°C, 3 min), plus a final polymerization step (72°C, 7 min). An approximate 700 bp fragment was amplified and cloned into pCR2.1-TOPO. Sequencing of the cloned fragment generated new 3' sequence data, including that which encoded the carboxyl terminus of the polypeptide.

Results from the preliminary sequencing described above were used to design oligonucleotide primers for the specific amplification of the *mhp3* gene directly from low-passage (n=4) *M. hyopneumoniae* strain 232 chromosomal DNA. These products were sequenced directly in an attempt to avoid the introduction of sequence artifacts due to possible mutations which may arise during PCR amplification and subsequent cloning steps.

Synthetic oligonucleotides which flank the *mhp3* gene were used to specifically amplify the open reading frame (ORF) from chromosomal DNA. PCR amplifications were carried out in triplicate and contained 1 µM each primer Mhp3-U1 (SEQ ID NO:31) and Mhp3-D (SEQ ID NO:33), 360 ng purified chromosomal DNA, 1x PC2 buffer (Ab Peptides, Inc; St. Louis, MO), 200 µM each dNTP, 7.5 U Klen*Taq*1 (Ab Peptides, Inc) and 0.15 U cloned *Pfu* (Stratagene; La Jolla, CA) thermostable polymerase in a 50 µL final sample volume. Conditions for amplification consisted of denaturation at 94°C for 5 min, followed by 25 cycles of denaturation (94°C, 30 sec), annealing (55°C, 30 sec), and polymerization (72°C; 3 min, 45 sec), plus a final extension at 72°C for 7 min. Following amplification, the triplicate sample was pooled and the specific product was purified by extraction with spin chromatography (QIAquick™ PCR purification kit, Qiagen; Santa Clarita, CA). The pooled mixture was then subjected to direct sequence analysis using DyeDeoxy termination reactions on an ABI automated DNA sequencer (Lark Technologies Inc., Houston, TX).

Synthetic oligonucleotide primers (SEQ ID NOs:16-28, 31, and 33) were used to sequence both DNA strands of the amplified products from *M. hyopneumoniae* strain 232. The nucleotide sequence of the *mhp3* gene and deduced Mhp3 protein encoded within this region is presented in SEQ ID NO:1 and Seq ID No 2, respectively.

The *mhp3* ORF extends from nucleotides 97-1452 of SEQ ID NO:1, and encodes a 451 amino acid protein (SEQ ID NO: 2) having a theoretical molecular weight of 49,775 daltons. The encoded polypeptide would contain 8 tryptophan residues, 7 which are encoded by the TGA codon (UGA in mRNA), known to specify for the addition of this amino acid in many mycoplasma spp. (Dybvig, K., 1990. Ann. Rev. Microbiol. 44:81-104; Yamao, F., Muto, A., Kawauchi, Y., 1985, Proc. Natl. Acad. Sci. U.S.A. 82:2306-2309). The amino terminus of the encoded protein appears to have properties characteristic of a prokaryotic signal sequence (von Heijne, G., 1985. J. Mol. Biol. 184: 99-105; Nielsen, H., Engelbrecht, J., Brunak, S., and von Heijne, G., 1997. Protein Engineering, 10: 1-6), although the precise site of cleavage is not presently known. The cysteine residue at position 29 of the encoded protein (SEQ ID NO: 2) is believed to be modified following processing by the addition of a sulfhydryl-linked fatty acid to make Mhp3 a lipoprotein (Razin, S., Yogev, D., and Naot, Y. 1998. Microbiol. Mol. Biol. Rev. 62:1094-1156).

When the *mhp3* ORF was compared against existing nucleotide and protein databases using the Basic Local Alignment Search Tool (BLAST) programs (Altschul, S.F., Gish, W., Miller, W., Myers, E.W., Lipman, D.J., 1990. J. Mol. Biol. 215:403-410), the entry with which it shared the greatest homology was the Ag 234-5 protein from *M*. *arginini* (GenBank Accession No. D16674). When these two polypeptides were aligned using CLUSTAL W (Thompson, J.D., Higgins, D.G., and Gibson, T.J., 1994. Nucl. Acids Res., 22:4673-4680), there is 36.2 % amino acid identity between the Mhp3 and Ag 234-5 proteins (Fig. 1). Though the gene encoding Ag 234-5 was originally identified as being present in *M. arginini* (Ushio, S., lwaki, K., Taniai, M, Ohta, T., Fukuda, S., Sugimura, K, and Kurimoto, M., 1995. Microbiol. Immunol. 39:395-400), recent evidence has revealed that this gene was actually derived from *M. hyorhinis* (Droesse, M., Wise, K.S., 1998. Abstract E20, 12^{th} International Organisation of Mycoplasmology Conference, Sydney, AU). In those studies, it was concluded that Ag 234-5 was not conserved among other mycoplasmas, including *M*. *hyopneumoniae,* as determined by PCR, Southern blot hybridization, and Western blot analysis.

Using the STEMLOOP program from the University of Wisconsin Genetics Computer Group (Devereux, J., Haeberli, P., and Smithies, O., 1984. Nuc. Acids Res. 12:387-395), a sequence was identified downstream of the *mhp3* ORF, specifically nucleotides 1519-1550 of SEQ ID NO:1, which could potentially form a stemloop structure. The stem could be formed by 14 base inverted repeats separated by a 4 base loop. The actual existence and potential function of this structure is presently unknown.

An additional ORF is also encoded by the DNA fragment represented in SEQ ID NO:5. This ORF is present on the opposite strand from gene *mhp3* and extends from nucleotide 602 to nucleotide 1 of SEQ ID NO:1 . This predicted ORF encodes a protein of at least 200 amino acids, designated "ORF1" and presented as SEQ ID NO:6, that has a theoretical molecular weight of 22,528 daltons. The fact that the ORF continues through the end of the DNA fragment (SEQ ID NO:1) and remains open suggests that the actual mass of the encoded polypeptide is likely greater than 22,528 daltons. Though Mhp3 and "ORF1" are encoded on opposite strands, the third base of each codon (the "Wobble" position) is common between them. Thus, the wobble base at any particular amino acid codon in "ORF 1" is shared by the opposite strand encoding a particular amino acid in the Mhp3 gene. This unique arrangement of shared coding sequences for two proteins should, in theory, minimize the impact of genetic drift at the codon wobble positions for Mhp3 and "ORF 1" and maximize the conservation of both encoded proteins.

### Preparation of Plasmid and Deposit Materials

The *mhp3* gene fragment was prepared for deposit with the American Type Culture Collection (ATCC). The pooled mixture resulting from triplicate PCR reactions employing specific 5' and 3' primers Mhp3-U1 (SEQ ID NO:31) and Mhp3-D (SEQ ID NO:33) as described *supra* was isolated by extraction with spin chromatography (QlAquick™) and inserted into the TA cloning site of pCR2.1-TOPO. Single sequence extension reactions utilizing vector-specific sequencing primers confirmed the endpoints of the amplified 1,692 base pair fragment, and revealed that the gene encoding Mhp3 was in the opposite orientation relative to the lactose promoter. This plasmid construct was designated pER427 and introduced into *E. coli* TOP10 cells (Invitrogen, Carlsbad, CA). The resulting strain was designated Pz427.

### Site-Directed Mutagenesis of the mhp3 Gene

The preparation of DNA encoding Mhp3 for expression in *E. coli* required modifying the *M. hyopneumoniae mhp3* gene by removal of the sequence that encodes the presumed leader and the fatty acid attachment site, cysteine 29, and the conversion of 6 TGA codons to TGG codons. The oligonucleotide primers used for the amplification of the gene lacking the sequence encoding for the signal peptide were designated RAC 23 (SEQ ID NO:29) and RAC 24 (SEQ ID NO:30). The conditions for amplification of that fragment from *M*. *hyopneumoniae* strain 232 (passage n=5) chromosomal DNA were denaturation at 94°C for 9 min, followed by 25 cycles of denaturation (94°C, 1 min), annealing (50°C, 1 min), and polymerization (72°C, 2min), plus a final polymerization step (72°C, 7 min). The amplified fragment was cloned into pCR2.1-TOPO; confirmation of cloning was by sequencing and restriction endonuclease digestion followed by gel electrophoresis. The sequence at the 5' end of the cloned fragment encodes for the initiating methionine residue, then a tryptophan residue, which is the amino acid following the cysteine residue at position 29 of the encoded polypeptide (see SEQ ID NO:2). At the 3' end, base changes were introduced into the wild-type nucleotide sequence (SEQ ID NO:1) which created a restriction site for cloning purposes. This resulted in the carboxy-terminal 2 amino acids of the wild-type polypeptide, Lys-Asn (SEQ ID NO:2) being replaced by the sequence Asn-Leu (see residues 422-423 in SEQ ID NO:4). An additional T nucleotide was present in the RAC 24 primer (SEQ ID NO:30) compared to the wild type gene, but was not present in the PCR product. Oligonucleotides RAC 23 (SEQ ID NO:29) and RAC 24 (SEQ ID NO:30) contained synthetic restrictions sites *Nde*l and *Xba*l*,* respectively, near their 5' ends to facilitate the cloning of the gene into various plasmids. Additional nucleotides (6 for RAC 23 (SEQ ID NO:29); 7 for RAC 24 (SEQ ID NO:30)) were also added at the 5' end of each primer to facilitate cutting by the respective restriction enzymes.

Once the sequence encoding for the signal peptide was removed, six internal TGA codons remained in the gene. To enable expression of the full-length polypeptide in *E. coli,* these were converted to TGG codons by oligonucleotide-directed *in vitro* mutagenesis. Plasmid pCR2.1-TOPO:*mhp*3 was transformed into *E*. *coli* strain CJ236 *(dut⁻ ung⁻).* The addition of helper phage R408 to cells containing the plasmid led to the production of phage particles having uracil-containing single-stranded (ss) DNA. These particles were isolated, and ssDNA was purified by extraction and precipitation. Mutagenesis was carried out using this DNA as template, oligonucleotides Mhp3-2M, Mhp3-3M, Mhp3-4M, Mhp3-5M, Mhp3-6M, Mhp3-7M SEQ ID NOs:35-40), and reagents from the MutaGene System (BioRad Laboratories; Hercules, CA). The ssDNA, oligonucleotides and annealing buffer were mixed and heated to 70°C, then allowed to cool to 30°C over a 1 hr period. Synthesis of the complementary strand was performed by adding T4 DNA ligase, T7 DNA polymerase, and synthesis buffer. The mixture was incubated on ice for 5 min, then room temperature for 5 min, followed by 37°C for 30 min. The resulting double-stranded DNA molecules were transformed into DH5α UltraComp cells (Gibco BRL; Gaithersburg, MD). Clones were propagated and screened for the desired mutations by sequencing. All mutations (TGA>TGG) were confirmed in this manner. In addition, an A>G transition inadvertently occurred corresponding to nucleotide 388 of the wild-type gene (SEQ ID NO:1) by virtue of the sequence of oligonucleotide Mhp3-2M (SEQ ID NO:35). This resulted in alteration of the encoded residue from serine(AGT) at amino acid residue 98 of wild-type Mhp3 (SEQ ID NO:2) to glycine (GGT) at amino acid residue 70 of recombinant Mhp3 (SEQ ID NO:4).

The recombinant *mhp3* gene (with or without the inadvertent A>G transition described *supra)* that resulted from the above changes is depicted in SEQ ID NO:3, and the open reading frame encoded by the recombinant *mhp3* gene (with glycine at amino acid residue 70) is depicted in SEQ ID NO:4.

### Cloning of Recombinant mhp3 Gene into Expression Vector and Expression Host Strain

For the purpose of recombinant protein expression, the mutated *mhp3* gene lacking the sequence encoding the signal peptide (SEQ ID NO:3) was cloned into the pBAD/Thio-TOPO expression plasmid (Invitrogen); this construct was directly transformed into the expression host *E*. *coli* BL21. A clone was identified which contained the appropriate plasmid.

### Expression of Recombinant Mhp3 Protein

Frozen working stock of the *E. coli* BL21 transformant expressing the thioredoxin-Mhp3 fusion was thawed and seeded at a 1:5000 dilution into RWLDM/D vi defined medium, which contains the following: K₂HPO₄ (6 g/L), KH₂PO₄ (3 g/L), (NH₄)₂SO₄ (5 g/L), NaCl (2 g/L), 0.2 mL CaCl₂ (15 g/L), 0.4 mL FeCl₃.6H₂O (5 g/L), 0.4 ml MgSO₄.7H₂O (480 g/L), ZnCl₂ (6.5 g/L), MnSO₄.H₂O (12 g/L), Na₂MoO₄.2H₂O (5 g/L), CuSO₄ (1.5 g/L), CoCl₂.6H₂O (2 g/L), H₃BO₃ (0.5 g/L), and 37% HCl (5ml/L). Carbenicillin was also added to a concentration of 125 µg/mL. The culture was grown under fed-batch conditions (50% dextrose) in a 5 liter working volume BioFlow 3000 fermentor (New Brunswick Scientific; Edison, NJ) at 37°C until A₆₂₅ was 10-20.

Wet cells of the *E. coli* BL21 transformant expressing recombinant thioredoxin-Mhp3 from the 5 liter fermentation were harvested by centrifugation and re-suspended in phosphate-buffered saline. The cells were mechanically lysed. Following centrifugation, the pellet was discarded. The supernatant was passed over an ion exchange column, and eluted off using a NaCl gradient. Fractions containing the fusion protein were pooled, dialyzed to remove the NaCl, and filter-sterilized using a 0.2 µm filter. This preparation is used for the vaccination trials.

### Immunological Characterization of Recombinant Mhp3

The protein concentration of the recombinant thioredoxin-Mhp3 prepared as described above was determined using a BCA Protein Assay kit (Pierce). In brief, each sample was diluted 1/10, 1/20, 1/40, and 1/80 in sterile deionized, distilled water (ddH₂O). BSA (protein standard) was diluted to concentrations ranging from 200 to 800 µg/mL. A 20 µL volume of sample or standard was added to triplicate wells in a 96 well microtiter plate, and 200µL of Reagent B diluted 1/50 in Reagent A was added to each well. The plate was incubated at 37°C for 30 min. Sample absorbance was determined at 560 nm. The protein concentration for each sample was calculated by extrapolation using the BSA standard curve.

Aliquots of recombinant Mhp3 (protein load was variable) and whole bacterial cell lysates of *M. hyopneumoniae, Mycoplasma hyorhinis,* and *Mycoplasma mycoides* subspecies *mycoides* were resuspended to a final volume of 10 µL, and 10 µL of 2x reducing sample buffer (Owl Scientific) was added. Samples were heated for 10 min. at 100°C, and the entire volume was loaded into separate wells of a 10-well, 1.5 mm thick, 10% Tris-glycine gel (Novex). Unstained, broad-range molecular weight markers (Novex) were also included.

Proteins separated by SDS-PAGE were transferred to PVDF membrane (Owl Scientific) at 100 mA constant current for 1 hr. The blot was incubated in blocking buffer consisting of 5% skim milk powder and 0.5% Tween 20 in TBS (TBST) for 1 hr at room temperature with gentle agitation. The blocking buffer was removed, and the membrane was washed 1 time for 5 min with TBST. The primary antibody was obtained from a pig following experimental challenge with *M. hyopneumoniae* strain 232. Diluted serum was added to the membrane, followed by a 1 hr incubation at room temperature. Alkaline phosphatase-conjugated Protein G antibody (Pierce) was diluted, added to the washed membrane, and incubated for 1 hr at room temperature. The membrane was washed with TBST, and the substrate BCIP/NBT (Kirkegaard and Perry Laboratories) was added to the membrane and incubated until a suitable color reaction developed. The membrane was then rinsed with water to stop the reaction, and dried at room temperature.

Serum from the pig experimentally challenged with *M. hyopneumoniae* did recognize the purified recombinantly-expressed Mhp3 (FIG. 2), as a band of 60kDa was identified. This suggests that the recombinant protein expressed epitopes which were recognized by antibodies generated in response to exposure of the pig to whole-cell *M. hyopneumoniae* organisms.

### Animal Study to Test Efficacy of Recombinant Mhp3 as a Vaccine Candidate

Healthy crossbred pigs (approximately 12 to 16 days of age) without a history of previous vaccination against *M. hyopneumoniae* or disease caused by *M. hyopneumoniae* are obtained. Animals are randomly assigned by litter into groups. Pigs are allowed to acclimate for a minimum of five days prior to the initiation of the study.

Animals are vaccinated with 1 mL of the appropriate experimental vaccine by the intramuscular route (IM; left neck muscle) on day "0" when pigs are approximately 19 to 23 days of age. At approximately two to three weeks following first vaccination pigs receive a second 1 mL dose (IM; right neck muscle) of the appropriate experimental vaccine. All pigs are closely observed (for up to 1 hour or as warranted) for any post-vaccinal signs such as vomiting, depression, diarrhea, ataxia-incoordination, increased respiration, or trembling.

At approximately two to four weeks following the second vaccination, pigs are challenged intranasally with 1 mL/nare of a live virulent lung homogenate culture of *M. hyopneumoniae* strain 232 containing approximately 5.0 X 10⁸ color changing units/mL (CCU/mL).

All challenged animals are necropsied at approximately 4 weeks following challenge. Lungs are removed and evaluated grossly for characteristic lesions attributable to a *M*. *hyopneumoniae* infection. Individual lung lesion scores will be determined by image analysis. A bias sample of lung tissue may be obtained from each challenged animal for bacterial isolation (CCU/g of tissue), histopathology, and IFA.

### DEPOSIT OF MICROORGANISMS

The following microorganism strain was deposited with the American Type Culture Collection (ATCC), 10801 University Blvd., Manassas, VA on September 9, 1999 and has been assigned the accession number indicated below. The depositor was Pfizer Inc. whose registered address is 235 East 42^{nd} Street, New York, New York 10017, United States.

| Microorganism | Accession Number |
|---|---|
| Pz427 | PTA-634 |

The present invention is not to be limited in scope by the specific embodiments described herein. Indeed, various modifications of the invention in addition to those described herein will become apparent to those skilled in the art from the foregoing description and accompanying drawing. Such modifications are intended to fall within the scope of the appended claims.

## Claims

1. A protein having an amino acid sequence comprising SEQ ID NO:4, wherein said protein does not have a fatty acid acylated cysteine followed by the amino acid sequence Trp Asp Lys Glu, and does not have a C-terminal homoserine lactone.

2. The protein of claim 1 which is an isolated protein.

3. The protein of claim 1 which is a fusion protein.

4. The protein of claim 3, in which the fusion protein is a thioredoxin fusion protein.

5. A composition comprising the protein of any one of claims 1-4, and a pharmaceutically acceptable carrier.

6. The composition of claim 5, further comprising an adjuvant.

7. The composition of claim 5 or 6, further comprising at least one polypeptide selected from the group consisting of *Mycoplasma hyopneumoniae* P46, P65, P97 and P102.

8. An immunogenic protein having an amino acid sequence as depicted in SEQ ID NO:4, wherein the immunogenic protein does not have a fatty acid acylated cysteine followed by the amino acid sequence Trp Asp Lys Glu, and does not have a C-terminal homoserine lactone.

9. Use of a protein as defined in any one of claims 1-4, in the manufacture of a vaccine formulation for treating or preventing a disease or disorder in an animal caused by infection with *Mycoplasma hyopneumoniae.*

10. The use of claim 9, wherein said animal is a pig.

11. A DNA encoding in the universal genetic code a protein having an amino acid sequence comprising SEQ ID NO:4, or its complement.

12. The DNA of claim 11 operably linked to a heterologous promoter.

13. The DNA of claim 12 which further comprises an origin of replication active in a prokaryotic cell.

14. The DNA of claim 12 which further comprises an origin of replication active in a eukaryotic cell.

15. A host cell comprising the isolated DNA of claim 12.

16. The host cell of claim 15, wherein said cell is *E. coli* BL21 and said DNA is the expression vector pBAD/Thio-TOPO.

17. A method for the production of the protein claimed in claim 1, said method comprising (i) growing the cells of claim 15 and 16 under conditions wherein said protein is expressed, and (ii) recovering said protein.

18. The method of claim 17, wherein said protein is recovered in a soluble form.

19. The method of claim 17, wherein said protein is recovered in an insoluble form.

20. Use of the DNA of claim 11 in the manufacture of a vaccine formulation for treating or preventing a disease or disorder in an animal caused by infection with *Mycoplasma hyopneumoniae.*

21. The use of claim 20 wherein said animal is a pig.

22. A kit suitable for diagnosis of *M*. *hyopneumoniae* infection comprising in at least one container a protein having an amino acid sequence comprising a protein as defined in claim 1.

23. The kit of claim 22, further comprising an anti-pig secondary antibody.

24. The kit of claim 23, in which the secondary antibody is conjugated to an enzyme that catalyzes a colorimetric reaction.

25. The kit of claim 24, wherein the enzyme is selected from the group consisting of alkaline phosphatase and horseradish peroxidase.

26. The kit of claim 24, further comprising reagents for a colorimetric assay.

## Patentansprüche

1. Protein mit einer Aminosäuresequenz, die SEQ ID NO:4 umfasst, wobei das Protein kein mit einer Fettsäure acyliertes Cystein hat, dem die Aminosäuresequenz Trp Asp Lys Glu folgt, und kein C-terminales Homoserin-Lacton hat.

2. Protein nach Anspruch 1, das ein isoliertes Protein ist.

3. Protein nach Anspruch 1, das ein Fusionsprotein ist.

4. Protein nach Anspruch 3, in dem das Fusionsprotein ein Thioredoxin-Fusionsprotein ist.

5. Zusammensetzung, umfassend das Protein nach einem der Ansprüche 1-4 und einen pharmazeutisch verträglichen Träger.

6. Zusammensetzung nach Anspruch 5, die weiterhin ein Adjuvans umfasst.

7. Zusammensetzung nach Anspruch 5 oder 6, weiterhin umfassend wenigstens ein Polypeptid, das ausgewählt ist aus der Gruppe, bestehend aus *Mycoplasma hyopneumoniae* P46, P65, P97 und P102.

8. Immunogenes Protein mit einer Aminsäuresequenz, wie sie in SEQ ID NO:4 dargestellt ist, wobei das immunogene Protein kein mit einer Fettsäure acyliertes Cystein hat, dem die Aminosäuresequenz Trp Asp Lys Glu folgt, und kein C-terminales Homoserin-Lacton hat.

9. Verwendung eines Proteins, wie es in einem der Ansprüche 1-4 definiert ist, bei der Herstellung einer Vaccinformulierung zur Behandlung oder Verhütung einer Erkrankung oder Störung bei einem Tier, die durch Infektion mit *Mycoplasma hyopneumoniae* verursacht wird.

10. Verwendung nach Anspruch 9, wobei das Tier ein Schwein ist.

11. DNA, die im universellen genetischen Code für ein Protein mit einer Aminosäuresequenz, die SEQ ID NO:4 umfasst, codiert, oder ihr Komplement.

12. DNA nach Anspruch 11 in funktioneller Verknüpfung mit einem heterologen Promotor.

13. DNA nach Anspruch 12, die weiterhin einen in einer prokaryotischen Zelle wirksamen Replikationsstartpunkt umfasst.

14. DNA nach Anspruch 12, die weiterhin einen in einer eukaryotischen Zelle wirksamen Replikationsstartpunkt umfasst.

15. Wirtszelle, umfassend die isolierte DNA nach Anspruch 12.

16. Wirtszelle nach Anspruch 15, wobei die Zelle *E. coli* BL21 ist und die DNA der Expressionsvektor pBAD/Thio-TOPO ist.

17. Verfahren zur Produktion des in Anspruch 1 beanspruchten Proteins, wobei das Verfahren umfasst: (i) Wachstum der Zellen nach Anspruch 15 und 16 unter Bedingungen, unter denen das Protein exprimiert wird und (ii) Gewinnung des Proteins.

18. Verfahren nach Anspruch 17, wobei das Protein in einer löslichen Form gewonnen wird.

19. Verfahren nach Anspruch 17, wobei das Protein in einer unlöslichen Form gewonnen wird.

20. Verwendung der DNA nach Anspruch 11 bei der Herstellung einer Vaccinformulierung zur Behandlung oder Verhütung einer Erkrankung oder Störung bei einem Tier, die durch Infektion mit *Mycoplasma hyopneumoniae* verursacht wird.

21. Verwendung nach Anspruch 20, wobei das Tier ein Schwein ist.

22. Kit, der zur Diagnose von *M. hyopneumoniae-*Infektion geeignet ist, der in wenigstens einem Behälter ein Protein mit einer Aminosäuresequenz umfasst, umfassend ein Protein wie es in Anspruch 1 definiert ist.

23. Kit nach Anspruch 22, der weiterhin einen Anti-Schwein-Sekundärantikörper umfasst.

24. Kit nach Anspruch 23, bei dem der Sekundärantikörper mit einem Enzym konjugiert ist, das eine colorimetrische Reaktion katalysiert.

25. Kit nach Anspruch 24, wobei das Enzym ausgewählt ist aus der Gruppe, bestehend aus alkalischer Phosphatase und Meerrettich-Peroxidase.

26. Kit nach Anspruch 24, der weiterhin Reagenzien für einen colorimetrischen Assay umfasst.

## Revendications

1. Protéine ayant une séquence d'acides aminés comprenant SEQ ID N°4, où ladite protéine ne comporte pas de cystéine acylée par un acide gras suivie par la séquence d'acides aminés Trp Asp Lys Glu, et ne comporte pas d'homosérine lactone C-terminale.

2. Protéine selon la revendication 1, qui est une protéine isolée.

3. Protéine selon la revendication 1, qui est une protéine de fusion.

4. Protéine selon la revendication 3, dans laquelle la protéine de fusion est une protéine de fusion thiorédoxine.

5. Composition comprenant la protéine selon l'une quelconque des revendications 1-4, et un support pharmaceutiquement acceptable.

6. Composition selon la revendication 5, comprenant en outre un adjuvant.

7. Composition selon la revendication 5 ou 6, comprenant, en outre, au moins un polypeptide sélectionné dans le groupe consistant en *Mycoplasma hyopneumoniae* P46, P65, P97 et P102.

8. Protéine immunogène ayant une séquence d'acides aminés telle qu'illustrée par SEQ ID N°4, où la protéine immunogène ne comporte pas de cystéine acylée par un acide gras suivie par la séquence d'acides aminés Trp Asp Lys Glu, et ne comporte par d'homosérine lactone C-terminale.

9. Utilisation d'une protéine telle que définie dans l'une quelconque des revendications 1-4, dans la fabrication d'une formulation de vaccin pour traiter ou prévenir, chez un animal, une maladie ou un trouble causé par une infection à *Mycoplasma hyopneumoniae.*

10. Utilisation selon la revendication 9, dans laquelle ledit animal est un porc.

11. ADN codant, dans le code génétique universel, une protéine ayant une séquence d'acides aminés comprenant SEQ ID N°4, ou son complément.

12. ADN selon la revendication 11, lié opérationnellement à un promoteur hétérologue.

13. ADN selon la revendication 12, qui comprend, en outre, une origine de réplication active dans une cellule procaryote.

14. ADN selon la revendication 12, qui comprend, en outre, une origine de réplication active dans une cellule eucaryote.

15. Cellule hôte comprenant l'ADN isolé selon la revendication 12.

16. Cellule hôte selon la revendication 15, dans laquelle ladite cellule est *E. coli* BL21 et ledit ADN est le vecteur d'expression pBAD/Thio-TOPO.

17. Procédé pour la production de la protéine revendiquée dans la revendication 1, ledit procédé comprenant (i) la culture des cellules selon la revendication 15 ou 16 dans des conditions dans lesquelles ladite protéine est exprimée et (ii) la récupération de ladite protéine.

18. Procédé selon la revendication 17, dans lequel ladite protéine est récupérée sous une forme soluble.

19. Procédé selon la revendication 17, dans lequel ladite protéine est récupérée sous une forme insoluble.

20. Utilisation de l'ADN selon la revendication 11 dans la fabrication d'une formulation de vaccin pour traiter ou prévenir, chez un animal, une maladie ou un trouble causé par une infection à *Mycoplasma hyopneumoniae.*

21. Utilisation selon la revendication 20, dans laquelle ledit animal est un porc.

22. Kit convenant au diagnostic d'une infection à *M. hyopneumoniae* comprenant, dans au moins un récipient, une protéine ayant une séquence d'acides aminés comprenant une protéine selon la revendication 1.

23. Kit selon la revendication 22, comprenant, en outre, un anticorps secondaire anti-porc.

24. Kit selon la revendication 23, dans lequel l'anticorps secondaire est conjugué à une enzyme qui catalyse une réaction colorimétrique.

25. Kit selon la revendication 24, dans lequel l'enzyme est sélectionnée dans le groupe consistant en une phosphatase alcaline et une peroxydase de raifort.

26. Kit selon la revendication 24, comprenant, en outre, des réactifs pour test colorimétrique.
